# EUROPEAN PATENT SPECIFICATION

(11) **EP 3 821 252 B1**
(45) Date of publication and mention of the grant of the patent: **07.06.2023**
(21) Application number: 19746438.1
(22) Date of filing: 09.07.2019
(51) Int. Cl.: G01N 33/574

(54) **A METHOD OF PREDICTING RESPONSE TO TREATMENT IN CANCER PATIENTS**
VERFAHREN ZUR VORHERSAGE DER REAKTION AUF EINE BEHANDLUNG BEI KREBSPATIENTEN
PROCÉDÉ DE PRÉDICTION DE RÉPONSE AU TRAITEMENT CHEZ DES PATIENTS ATTEINTS D'UN CANCER

(30) Priority: 10.07.2018 GB 201811291
(43) Date of publication of application: 19.05.2021
(73) Proprietor: Dublin City University, Dublin 9 (IE)
(72) Inventor: COLLINS, Denis, Dublin 9 (IE); GAYNOR, Nicola, Dublin 9 (IE); CROWN, John, Dublin 9 (IE); O'DONOVAN, Norma, Dublin 9 (IE)
(74) Representative: Purdylucey Intellectual Property
(86) International application number: PCT/EP2019/068365
(87) International publication number: WO 2020/011770

(56) References cited:
- BEANO ALESSANDRA ET AL: "Correlation between NK function and response to trastuzumab in metastatic breast cancer patients", JOURNAL OF TRANSLATIONAL MEDICINE, BIOMED CENTRAL, vol. 6, no. 1, 16 May 2008 (2008-05-16), page 25, XP021037671, ISSN: 1479-5876
- RODNEY J. TAYLOR ET AL: "Ex Vivo Antibody-Dependent Cellular Cytotoxicity Inducibility Predicts Efficacy of Cetuximab", CANCER IMMUNOLOGY RESEARCH, vol. 3, no. 5, 1 May 2015 (2015-05-01), pages 567-574, XP055624964, US ISSN: 2326-6066, DOI: 10.1158/2326-6066.CIR-14-0188
- A. M. TROTTA ET AL: "Prospective Evaluation of Cetuximab-Mediated Antibody-Dependent Cell Cytotoxicity in Metastatic Colorectal Cancer Patients Predicts Treatment Efficacy", CANCER IMMUNOLOGY RESEARCH, vol. 4, no. 4, 1 April 2016 (2016-04-01), pages 366-374, XP055624971, US ISSN: 2326-6066, DOI: 10.1158/2326-6066.CIR-15-0184
- FISCHER L ET AL: "The anti-lymphoma effect of antibody-mediated immunotherapy is based on an increased degranulation of peripheral blood natural killer (NK) cells", EXPERIMENTAL HEMATOLOGY, ELSEVIER INC, US, vol. 34, no. 6, 1 June 2006 (2006-06-01), pages 753-759, XP027879574, ISSN: 0301-472X [retrieved on 2006-06-01]
- MARIA LILJEFORS ET AL: "Natural killer (NK) cell function is a strong prognostic factor in colorectal carcinoma patients treated with the monoclonal antibody 17-1A : NK Cells in Antibody Therapy", INTERNATIONAL JOURNAL OF CANCER, vol. 105, no. 5, 10 July 2003 (2003-07-10) , pages 717-723, XP055625171, US ISSN: 0020-7136, DOI: 10.1002/ijc.11139
- BRANKA PETRICEVIC ET AL: "Trastuzumab mediates antibody-dependent cell-mediated cytotoxicity and phagocytosis to the same extent in both adjuvant and metastatic HER2/neu breast cancer patients", JOURNAL OF TRANSLATIONAL MEDICINE, BIOMED CENTRAL, vol. 11, no. 1, 12 December 2013 (2013-12-12), page 307, XP021171152, ISSN: 1479-5876, DOI: 10.1186/1479-5876-11-307
- K. H. YU ET AL: "Pharmacogenomic Modeling of Circulating Tumor and Invasive Cells for Prediction of Chemotherapy Response and Resistance in Pancreatic Cancer", CLINICAL CANCER RESEARCH, vol. 20, no. 20, 8 August 2014 (2014-08-08), pages 5281-5289, XP055231872, US ISSN: 1078-0432, DOI: 10.1158/1078-0432.CCR-14-0531

## Description

### Field of the Invention

The current invention relates to a method of predicting or determining response to treatment in cancer patients, in particular, in HER2+ cancer patients, for example, breast cancer patients.

### Background of the Invention

Globally, in 2019, it is estimated that 268,000 women will be diagnosed with breast cancer and it is estimated that 41760 women will die from breast cancer. Each year, around 2,800 women are diagnosed with invasive breast cancer in Ireland and around 700 women die of the disease. Many therapies exist but innate and developed resistance to these therapies is a major issue in breast cancer treatment. Prescribing a particular treatment to a patient who is unlikely to respond appropriately is not desirable for many reasons, such as severe side effects, high cost and delay to appropriate treatment. Therefore, there is a clinical need for the development of more patient-specific treatments to improve patient outcome. In this way, patients who have the best chance of responding are treated quickly and effectively and non-responders are not unnecessarily treated and an alternative treatment strategy can be pursued.

Breast cancer (BC) cells have growth-stimulating receptors on their surface and in their cytoplasm. These receptors are used to classify breast cancer types and include, among others, estrogen receptor (ER), progesterone receptor (PR) and human epidermal growth factor 2 (HER2). Some breast cancer cells have no clinically targeted receptors and this type of cancer is called triple negative (TN) breast cancer.

HER2 is a protein that is overexpressed in approximately 20-25% of breast cancers. HER2-positive (HER2+) breast cancer has a number of approved therapies, including, targeted monoclonal antibody (mAbs) and small molecule therapies, administered alone or in combination with chemotherapy. Trastuzumab (Herceptin^{®}) is an antibody therapy that targets HER2. It is used in combination with chemotherapeutic drugs, e.g. Docetaxel (Taxotere^{®}) and carboplatin, as a first line treatment or alone in patients with metastatic disease. Trastuzumab (Herceptin) is not currently used to treat other types of breast cancer, such as TN or ER-positive breast cancer.

Trastuzumab binds to HER2, which is a receptor on the tumour surface and acts by inhibiting intracellular signalling and by generating an antibody-dependent cell-mediated cytotoxicity (ADCC) response against the tumour cell whereby immune cells, such as natural killer (NK) cells, bind trastuzumab and kill the tumour cells.

There are various mechanisms by which an immune cell can kill a cancer cell (immune cell-mediated cytotoxicity). Certain immune cell populations can recognise and kill cancer cells directly (direct immune cell-mediated cytotoxicity). Another mechanism by which immune cells can kill cancer cell is antibody-dependent (ADCC). ADCC is a normal effector cell-mediated process of immune response within the body relying on antibodies generated against specific antigens which are part of the humoral immune response (which also includes complement proteins and anti-microbial peptides). These antibodies bind to antigens on the surface of cells that are recognised as foreign, such as tumour cells. An effector cell of the immune system, such as a natural killer (NK) cell, interacts with the antibodies through Fc receptors causing the NK cell to release cytotoxic factors and lyse the target cell. Macrophages, eosinophils, mast cells and neutrophils can also mediate ADCC. Macrophages can also engage the antibodies in antibody-dependent cell mediated phagocytosis (ADCP). ADCC-capable antibody therapies such as trastuzumab can function as surrogates for antibodies produced by the body, allowing immune cells capable of killing the tumour cells in the presence of anti-tumour antibody to do so. The ability of certain molecules to elicit ADCC is well defined and studied as a therapeutic strategy. Antibody therapies can be engineered to have enhanced ADCC and newer technologies such as fusion proteins and bi- and tri-specific molecules that target specific antigens can be designed to exploit the biological process of ADCC (Zahavi et al., 2018 Antibody Therapeutics 1:1,7-12, Rath et al., 2015 PMID: 24156398, Schmohl et al., 2016 PMID: 27157665).

Trastuzumab is well characterised as an ADCC capable molecule. 10 to 15 years ago it was suspected that the ADCC response to trastuzumab in the lab using circulating immune cells from patients could be used as an indicator of patients who would respond well to trastuzumab-containing chemotherapy. There were also several groups reporting conflicting results. Boero *et al.,* 2015, PMID: 26450443 (trastuzumab) and Lo Nigro *et al.,* 2016 PMID: 26909137 (cetuximab) discuss a correlation between circulating immune cells' ADCC ability and response to therapy but the numbers were generally small (<45 pts). Botticelli *et al.,* 2015 PMID: 28983344, reported no association between polymorphisms (naturally occurring variations in Fc receptors with varying ability to elicit ADCC) and response to trastuzumab. Hurvitz *et al,* 2012 PMID: 22504044 describes a study involving 1189 patients and reported no link between Fc receptor polymorphisms and response to trastuzumab. This large study indicated that the strength of the ADCC response to trastuzumab was not important as the patients with immune cells with low ADCC capacity polymorphisms had the same response to trastuzumab as patients with high ADCC capacity polymorphisms. While this did not rule out a role for ADCC in trastuzumab function, if the level of ADCC response was critical to the clinical response to trastuzumab, the patients with low ADCC capacity immune cells should have had a worse outcome. This was not the case.

Approximately 40% of HER2+ patients receiving HER2-targeted therapy/chemotherapy in the neo-adjuvant setting will achieve a pathological complete response (Basho and McArthur 2018 PMID: 30034547. Currently, there is no test, ADCC-related or otherwise, to determine which HER2+ breast cancer patients will have a complete response to chemotherapy/trastuzumab treatment.

Immune checkpoint inhibitor (ICI)-based therapies (anti-CTLA-4, anti-PD-1 and anti-PD-L1) are currently under early clinical investigation in HER2+ and TN- breast cancer subtypes. ICI therapies, such as ICI antibody therapies, are therapies that target proteins, referred to as checkpoint proteins, expressed on immune cells (or in some instances on tumour cells) that are related to a suppressed anti-tumour immune response. It is known in the art that by inhibiting these checkpoint proteins, the anti-tumour immune response can be restored. The most success for ICls as monotherapies to date has been in melanoma (20-40% response rates, Jessurun *et al.,* 2017 PMID: 29034210), lung cancer (14%-75%, proportional to tumour PD-L1 expression, Aguiar PN *et al.,* 2017 PMID: 29209522). and kidney cancer (12%-40%, Ghatalia, P et al., 2017 PMID: 28210995) There are no ICls currently approved for the treatment of breast cancer. However, the ICI, avelumab and atezolizumab, are showing promise in early breast cancer treatment clinical trials. Pembrolizumab and trastuzumab has shown activity and durable clinical benefit in PD-L1 positive, advanced HER2+ breast cancer patients in an early Phase Ib/II trial (Loi et al., 2019 PMID: 30765258).

CD8+ T cells are the effectors of the adaptive immune response. They target and kill tumour cells directly as they recognise specific parts of the tumour cell that are different to normal tissue. It is at this stage in the response that immune checkpoints can become an issue in a patient. The tumour can create an environment that "exhausts" the CD8+ T cells so that the CD8+ T cells don't kill the tumour cells. To elicit this effect, tumour cells over-express checkpoint proteins, e.g. PD-L1, or secrete factors that induce checkpoint proteins in immune cells. These proteins send out a "don't kill" message to the patient's immune system. ICIs function to inhibit these checkpoint proteins and increase the anti-tumour response (Pardoll 2012 PMID: 22437870).

Multiple approaches are now being pursued to design therapies that enhance the immune response against cancer by targeting modulators of the immune response. Negative regulators include the PD-1/PD-L1 axis, CTLA4, KIR, IDO, and A2AR and further approaches now focus on activating positive regulators of the immune response on the same immune cell populations, for example 4-1BB, OX-40 and CD137 [Marin-Acevedo 2018 PMID: 29544515]. The cytotoxicity of an immunosuppressed immune cell can therefore be enhanced using a range of different immune response modulators, depending on the mechanism of suppression at work.

Like trastuzumab, ICIs do not work well for every patient. The reasons for this are two-fold: sometimes a patient's immune system has not developed a response to the tumour or the immune system has developed a response but the ICls are ineffective as the immune system is being suppressed through other unrelated mechanisms. Those tumours that respond to ICls are generally referred to as "hot" while those that do not respond are termed "cold". The most extensively studied and most developed ICls currently target three checkpoint proteins: CTLA-4 (e.g. ICI Ipilimumab or Tremelimumab), PD-1 (e.g. ICI Pembrolizumab or Nivolumab) and PD-L1 (e.g. ICI Atezolizumab, Durvalumab or Avelumab). Most ICls including Pembrolizumab cannot elicit an ADCC response, whereas the ICls Avelumab and Ipilimumab are capable of eliciting an ADCC response against cancer cells lines (Boyerinas *et al.,* 2015 PMID: 26014098, Laurent *et al.,* 2013 PMID: 23634660).

Multiple approaches are being pursued to identify biomarkers of response, with a liquid biopsy (blood sample-based) option remaining the most sought.

The best method currently available involves testing patient tumours for the levels of PD-L1 to indicate if the tumour is part of an "exhausted" environment. 45% of cancer patients with high PD-L1 expression respond to ICls, however, 15% of patients without PD-L1 in their tumours still respond to ICls. Therefore, there is a need for new approaches and assays to stratify patients for ICI therapy (Sunshine and Taube 2015 PMID: 26047524).

WO2016007235 relates to PD-L1 antibodies and methods of using same. It discloses that PD-L1 is expressed in many cancers and that it has a role in immune system regulation. The authors focus on detecting PD-L1 in a biological sample and stated that the presence or expression of PD-L1 in the sample, using an antibody, indicated that the subject is likely to respond to treatment with an anti-cancer therapy. This document also suggests treatment of cancer with PD-1 axis binding antagonist. There is no disclosure regarding the effect of anti-PD-L1 or anti-PD-1 ICI on ADCC response or that this could be used as a biomarker for chemotherapy response.

Some groups, such as the following publications, have explored prediction of response to therapy in cancer patients. However, ICI-responsive ADCC as a biomarker of response to chemotherapy is not disclosed.

Beano Alessandra et al (Journal of Translational Medicine, Biomed Central, vol 6, no. 1, 16 May 2008) analysed a correlation between natural killer (NK) function and response to trastuzumab in metastatic cancer patents. The authors disclose assessment of ADCC against trastuzumab-coated HER2 positive cells in a sample from a patient receiving trastuzumab chemotherapy.

Rodney J Taylor et al (Cancer Immunology research, vol 3, no. 5, 1 May 2015) explores ADCC and prediction of efficacy of cetuximab. The authors use a method involving squamous cell carcinoma of the head and neck (SCCHN) cells or colon cancer calls and purified natural killer cells from patients. The cells are incubated with cetuximab, which is an ADCC capable molecule.

A.M Trotta et al, (Cancer Immunology Research, vol. 4, no. 4, 1 April 2016) discloses a test of 96 patients with metastatic colorectal cancer and assessed the FcγR status and cetuximab mediated ADCC. PBMC from patients were isolated at diagnosis and lymphokine-activated killer (LAK) generated. An NK cytotoxicity assay is also disclosed with an erythroleukemia cell line as a target cell incubated with LAK. The authors conclude that cetuximab-ADCC correlates with FcγR polymorphism.

Fischer L et al (Experimental Hematology, vol. 34, no. 6, 1 June 2006) discloses an assay that detects CD107a as a marker for natural killer cell degranulation to characterise peripheral blood natural killer cell mediating ADCC. The authors found an increase in natural killer cell degranulating in response to lymphoma cell line in patients treated with rituximab.

Maria Liljefors et al., (International Journal of Cancer, vol. 105, no. 5, 10 July 2003 investigated natural killer cell function as a prognostic factor in colorectal carcinoma patents treated with monoclonal antibody 17-1A.

Petricevic B, et al., ("Journal of Translational Medicine", 2013) describes a method to determine antibody-dependent cell medicated phagocytosis (ADCP) activity of PBMCs.

There is a current need to identify patients who will benefit from particular treatment methods and those who will not. The current invention provides a solution to this problem.

### Summary of the Invention

The Applicant has discovered that the ADCC response induced by a patient's blood cells against a cancer cell line and mediated by an ADCC-capable molecule, such as an ADCC-capable antibody therapeutic, in combination with an immune response modulator, such as an immune checkpoint inhibitor (ICI), can be used to predict the patient's response to cancer therapy, including chemotherapeutic regimens containing a targeted ADCC-capable antibody. In particular, a higher ADCC response to an ADCC-capable molecule in the presence of an immune response modulator, such as an ICI, compared with the reference response in the absence of an immune response modulator, is predictive of a poor response to cancer therapy, especially cancer therapy regimens containing targeted ADCC-capable antibody.

The method involves incubating a sample of the patient's blood with an ADCC-capable molecule (i.e, trastuzumab), an immune response modulator, and a cancer cell line that expresses the target of the ADCC-capable molecule (e.g. HER2+ cancer cell line). An ADCC response of the patient's blood cells to the ADCC-capable molecule and immune response modulator is determined, and then correlated with the reference value in the absence of the immune response modulator to predict whether the individual will respond to treatment with cancer therapy including those regimens containing ADCC-capable antibody therapies. The reference value may be an absolute value, or a relative value, or may be the ADCC response of the cancer cell line to the ADCC-capable molecule in the absence of the immune response modulator. Generally, the patient will have a cancer at the time of the test, typically a primary cancer, and it may be applied in the context of adjuvant therapy, metastatic or neoadjuvant therapy.

The invention is as set out in the appended claims. In a first aspect, the invention provides a method of predicting response to chemotherapy in a patient with cancer, the method comprising the steps of incubating an ADCC-capable molecule, an immune checkpoint protein modulator, a cancer cell line that expresses a target of the ADCC-capable molecule, and a sample of the patient's blood, for a period of time, determining an ADCC response against the cancer cell line in the presence of said immune checkpoint protein modulator, comparing the ADCC response with a reference ADCC response in the absence of an immune checkpoint protein modulator and predicting the patient's response to chemotherapy based on the comparison. Typically, the patient has a cancer, for example a primary cancer.

In one example, the chemotherapy is ADCC-capable antibody containing chemotherapy

In one embodiment, the reference ADCC response is determined by incubating the ADCC-capable molecule, the cancer cell line, and the sample of the patient's blood, without the immune checkpoint protein modulator, for a period of time, and determining an ADCC response against the cancer cell line to provide the reference ADCC response. Generally, a test ADCC response that is higher than a reference ADCC response, is indicative that the patient will have a poor response to chemotherapy, especially ADCC-capable antibody containing chemotherapy regimen.

In one embodiment, an elevated ADCC response in the immune response modulator-containing sample is predictive of a poor response to chemotherapy. In one embodiment, an elevated ADCC response to the immune checkpoint protein modulator containing sample is predictive of a poor response to chemotherapy comprising an ADCC-capable antibody. In one embodiment, an elevated ADCC response to the ICI containing sample is predictive of response to chemotherapy comprising an ICI.

In one embodiment, the method comprises the steps of:
incubating a first aliquot of the patient's blood cells with the ADCC-capable molecule, the immune checkpoint protein modulator, and the cancer cell line for a period of time to provide a first sample;
determining an ADCC response of the first sample;
incubating a second aliquot of the patient's blood sample with the ADCC-capable molecule and the cancer cell line in the absence of the immune checkpoint protein modulator to provide a second sample; and
determining an ADCC response of the second sample,
and when the ADCC response of the first sample is higher than the ADCC response of the second sample, predicting that the patient will have a poor response to chemotherapy, especially regimens containing ADCC-capable molecule targeted therapies.

In another embodiment, the method comprises the steps of:
incubating a first aliquot of the patient's blood sample with the ADCC-capable molecule, the checkpoint protein modulator, and the cancer cell line for a period of time to provide a first sample;
determining an ADCC response of the first sample;
incubating a second aliquot of the patient's blood sample with the ADCC-capable molecule and the cancer cell line in the absence of the immune checkpoint protein modulator to provide a second sample; and
determining an ADCC response of the second sample,
and when the ADCC response of the first sample is higher than the ADCC response of the second sample, predicting that the patient will respond to chemotherapy comprising an ICI.

In an embodiment, the cancer is any HER2+ or HER2 over-expressing cancer. Preferably, the cancer is HER2+ breast cancer. Other HER2+ or HER2 over-expressing cancers applicable to the present invention include, but are not limited to, gastric, oesophageal, gastro-oesophageal, ovarian, bladder, cervical, salivary gland, endometrial, pancreatic, colo-rectal, prostate, testicular, melanoma, hepatocellular carcinoma, cholangiocarcinoma, head and neck, small intestine, gall bladder and non-small-cell lung cancer (NSCLC). Such cancers are well known in the art.

In an embodiment, the cancer is any cancer. It may be HER2 negative. It may be HER2 low. The cancer may be, but are not limited to, gastric, oesophageal, gastro-oesophageal, ovarian, bladder, cervical, salivary gland, endometrial, pancreatic, colo-rectal, prostate, testicular, melanoma, hepatocellular carcinoma, cholangiocarcinoma, head and neck, small intestine, gall bladder and non-small-cell lung cancer (NSCLC).

In one embodiment, the chemotherapy comprises an ADCC-capable molecule. For example, an ADCC capable antibody therapy. In another embodiment, the chemotherapy comprises a chemotherapeutic selected from an alkylating agent, an anti-metabolite, an anti-microtubule agent, a topoisomerase inhibitor, and a cytotoxic antibiotic, optionally in combination with an ADCC-capable molecule. In one embodiment, the chemotherapy comprises docetaxel and/or carboplatin and/or trastuzumab. In one embodiment the chemotherapeutic comprises docetaxel and carboplatin combination.

In an embodiment of the method, the ADCC-capable molecule may be an ADCC-capable antibody, or antibody therapeutic.

In an embodiment of the method, the blood sample is a PBMC sample. The PBMC sample or fraction is isolated from a blood sample from a patient. Preferably, the PBMC fraction is isolated by density centrifugation. Typically, the PBMC fraction is isolated within four hours of blood draw from the patient. However, it will be appreciated that it may be isolated at any time after blood draw from the patient.,

In a preferred embodiment, the blood sample is one that has been isolated or taken from a patient before treatment for cancer commences. In an embodiment, the blood sample may be taken after treatment has initiated. In an embodiment, the blood sample is one that has been isolated or taken from a patient during treatment for cancer. It may be isolated, or taken, at any stage during treatment.

In an embodiment of the method, the amount of the ADCC capable antibody, such as trastuzumab, is 0.01-100, 0.1-100, 1-100, 1-30, 1-20 or 1-10 µg/ml.

In an embodiment of the method, the amount of an ICI is 0.01-100, 0.1-100, 1-100, 1-30, 1-20 or 1-10 µg/ml.

In an embodiment of the method, the immune checkpoint protein modulator is an immune checkpoint inhibitor (ICI).

In an embodiment, the ICI is a non-ADCC-capable ICI. The ICI may be an antibody. The ICI may be selected from the group comprising pembrolizumab, nivolumab, atezolizumab, pidilizumab, sintilimab, camrelizumab, spartalizumab, cemiplimab, tremelimumab, JS001, tislelizumab, durvalumab, MEDI0680, LY3300054 and FAZ053. Preferably, the ICI is pembrolizumab. The ICI may be an ADCC-capable ICI. The ICI may be AMP-224, AGEN-1884, avelumab or ipilimumab. The ICI could be a combination of one or more of any one of the ICls disclosed herein.

Preferably, the cancer cell line is matched with the cancer in the patient. Thus, for example, if the patient has a HER2+ breast cancer, the method of the invention will employ a HER2+ cancer cell line, preferably a HER2+ breast cancer cell line, Typically, the HER2+ cancer cell line, is a suitable breast cancer cell line (SKBR3). Any HER2+ or HER2 over-expressing cancer cell line, e.g. breast, gastric, ovarian, bladder or any other HER2 positive tumour indication, may be used. The cancer cell line may not be matched with the cancer in the patient, i.e. an unmatched cell line. Thus, for example, if the patient has a HER2+ breast cancer, the method of the invention may employ any cancer cell line, such as a lung cancer cell line, provided the cell line expresses a target of the ADCC capable molecule being utilised in the method. As a non-limiting example, the method may employ a blood sample from a patient with breast cancer, an ADCC capable molecule which is an antibody to c_MET and a lung cancer cell line that expresses c_MET and an ICI.

In an embodiment of the method, the PBMC may be counted and checked for viability before an amount of the ADCC capable molecule, e.g. trastuzumab and/or an immune response modulator, e.g. ICI, are added.

In an embodiment of the current method, a statistically significant difference in ADCC response between the first and second fraction indicates a greater chance of a poor response to chemotherapy. For example, a value of <0.05 indicates a greater chance of poor response to chemotherapy.

Disclosed herein and not claimed is a method of treating a patient with cancer comprising a step of predicting whether the patient will respond to chemotherapy using a method of the invention and, where it is predicted that the patient will have a greater chance of response to chemotherapy, administering chemotherapy to the patient, or optionally where it is predicted that the patient will not respond to chemotherapy, administering an ICI therapy to the patient.

Disclosed herein and not claimed is a method of treating a patient with cancer comprising a step of predicting whether the patient will respond to targeted ADCC-capable antibody containing regimens using a method of the invention and, where it is predicted that the patient will have a greater chance of response to targeted ADCC-capable antibody containing chemotherapy regimens, administering targeted ADCC-capable antibody containing regimens to the patient, or optionally where it is predicted that the patient will not respond to ADCC-capable antibody containing chemotherapy regimens, administering an ICI therapy to the patient.

In an aspect of the invention there is provided, a method of predicting response to chemotherapy in a patient with cancer, the method comprising the steps of:
Incubating an immune checkpoint protein modulator, a cancer cell line, and a sample of the patient's blood, for a period of time,
Determining direct cytotoxicity against the cancer cell line in the presence of an immune checkpoint protein modulator, comparing the direct cytotoxicity with a reference direct cytotoxicity in the absence of an immune checkpoint protein modulator, and predicting the patient's response to chemotherapy based on the comparison.

In one embodiment, the reference direct cytotoxicity is determined by incubating the cancer cell line, and the sample of the patient's blood, without the immune response modulator, for a period of time, and determining direct cytotoxicity against the cancer cell line to provide the reference direct cytotoxicity. Generally, a test direct cytotoxicity that is higher than a reference direct cytotoxicity, is indicative that the patient will have a poor response to chemotherapy.

All of the embodiments and preferred features of the method of the invention as disclosed herein are also applicable to this aspect of the method.

. In an embodiment of the method which determines direct cytotoxicity, the cancer cell line may be a HER2-low, HER2 negligible or HER2 negative cell line. Such cell lines are known in the art.

### Definitions

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and vice versa. The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein an "ADCC-capable molecule" refers to a molecule that is capable of binding to a cell surface protein, or antigen, such as a receptor, on the tumour surface, and generating an antibody-dependent cell-mediated cytotoxicity (ADCC), or antibody-dependent cell mediated phagocytosis (ADCP) response, against the tumour cell whereby immune cells, such as natural killer (NK) cells, bind the molecule and kill the tumour cell. Examples include but are not limited to ADCC capable antibodies, including IgG, IgE, and IgA antibodies and antibody fragments, or ADCC-enhanced IgG, IgE, and IgA antibodies (e.g. afucosylated, mutated) or Fc fusion proteins or artificial scaffolds or multi-specific (bi, tri, quad and beyond) constructs against tumour-specific and tumour-associated antigens. The molecule may be a mixture or combinations of ADCC capable molecules. The ADCC capable molecule includes one that has been altered or modified to produce a greater ADCC response than the unmodified molecule. It may be modified by methods known in the art such as afucosylation and mutation. Methods of determining ADCC are known in the art and also described herein.

As used herein, the term "ADCC-capable antibody" refers to an antibody, generally a therapeutic monoclonal antibody, capable of binding to a cell surface protein or antigen, such as a receptor, on the tumour surface and generating an antibody-dependent cell-mediated cytotoxicity (ADCC) or antibody-dependent cell-mediated phagocytosis (ADCP) response against the tumour cell whereby immune cells, such as natural killer (NK) cells, bind the antibody and kill the tumour cells. An example includes trastuzumab, which is specific for the HER2 tumour receptor in HER2+ breast and gastric cancers. Other ADCC-capable antibodies include, but are not limited to, the therapeutic antibodies pertuzumab (Perjeta ^{®}) which targets HER2 and is approved for the treatment of HER2+ breast cancer, Margetuximab which targets HER2 and is in clinical trials for the treatment of HER2+ breast cancer, gastric cancer and oesophageal cancer, cetuximab (Erbitux^{®}) which targets EGFR and is indicated for the treatment of EGFR-expressing WT KRAS colon cancer, EGFR-expressing non-small cell lung cancer and head and neck cancer. Rituximab (Rituxan ^{®}) targets CD20 and is indicated for the treatment of non-Hodgkin's lymphoma and chronic lymphocytic. leukemia Other examples of antibody therapies with and without ADCC capability can be found at https://www.mycancergenome.org/content/molecular-medicine/overview-of-targeted-therapies-for-cancer/ or in Shepard et al., 2017 PMID: 28572223.. The ADCC-capable antibody includes a mixture or combination of ADCC capable antibodies. The ADCC-capable includes one that has been altered or modified to produce a greater ADCC response than the unmodified antibody. It may be modified by methods known in the art such as afucosylation and mutation. The ADCC-capable antibody includes antibody fragments, or Fc-fusion proteins. Methods of determining ADCC are known in the art and also described herein.

Any ADCC-inducing IgG, IgE, and IgA antibodies against tumour specific and tumour associated antigens also fall under the definition of "ADCC-capable antibody".

As used herein, the term "cancer cell line" refers to an immortalized cancer cell line that expresses a tumour marker targeted by the ADCC-capable antibody. The term "cancer cell line" also includes primary cell lines. The cell line may be from the same patient that the blood sample is taken from or may be from a different patient. Generally, when the method of the invention is directed to prediction of response to a specific type of cancer in a patient, a cancer cell line having the same phenotype as the patient cancer can be employed. This may be referred to as a matched cell line. For example, if the patient has a HER2+ breast cancer, then the method of the invention will employ a HER2+ cancer cell line, preferably a HER2+ breast cancer cell line. Generally, cancer cells are used at a maximum concentration of 2.5 × 10e5 cells/ml. The cancer cell line may have a different phenotype as the patient cancer. This may be referred to as an unmatched cell line.

As used herein, the term "ADCC response" means a measure of the percentage of dead cells attributable to the agent added to the cancer cell line, i.e. an ADCC-capable molecule alone, or an ADCC-capable molecule and an ICI. Methods of measuring an ADCC response are known in the art and described below, and it will be appreciated that any such method may be used. Preferably, the ADCC response is measured by a flow cytometry-based ADCC assay. Other ADCC assay systems include but are not limited to LDH-based assays, chromium release assays, calcein -AM assays, high content analysis-based assays, luciferase reporter-based assays and Xcelligence^{®}-based assays. In one embodiment, the parameter of ADCC response is % cytotoxicity as determined using the method described below.

As used herein, the term "cancer therapy" or "chemotherapy"" should be understood to include, but are not limited to, cytotoxic chemotherapeutics (for example an alkylating agent, an anti-metabolite, an anti-microtubule agent, a topoisomerase inhibitor, and a cytotoxic antibiotic, or combinations thereof), targeted therapies (antibody therapy such as treatment with an ADCC capable antibody, immune based anti-body therapy, antibody-drug conjugates, small molecule inhibitors and small molecule tyrosine kinase inhibitors or a receptor modulator or disruptor), immune checkpoint inhibitor (ICI) based therapy and immune stimulatory based therapies

Examples of monoclonal antibody-targeted therapies include, but are not limited to, trastuzumab, which is specific for the HER2 tumour receptor in HER2+ breast and gastric cancers, the trastuzumab biosimilar trastuzumab-QYYP (Trazimera ^{®}), pertuzumab (Perjeta ^{®}) which targets HER2 and is approved for the treatment of HER2+ breast cancer, Margetuximab which targets HER2 and is in clinical trials for the treatment of HER2+ breast cancer, gastric cancer and oesophageal cancer, cetuximab (Erbitux^{®}) which targets EGFR and is indicated for the treatment of EGFR-expressing WT KRAS colon cancer, EGFR-expressing non-small cell lung cancer and head and neck cancer, Rituximab (Rituxan ^{®}) targets CD20 and is indicated for the treatment of non-Hodgkin's lymphoma and chronic lymphocytic.leukemia, and any ADCC-inducing IgG, IgE, and IgA antibodies against tumour specific and tumour associated antigens.

Examples of antibody drug conjugates include, but are not limited to, T-DM1 (HER2-targeting emtansine conjugate) used to treat HER2+ breast cancer and gemtuzumab ozogamicin (CD33-targeting calicheamicin conjugate) used to treat CD33-positive acute myeloid leukemia patients.

When used herein an "immune response modulator" refers to a molecule that is capable of controlling or altering the anti-cancer response, or cytotoxic response, of immune cells. One example of an immune response modulator is an immune checkpoint inhibitor (ICI).The immune response modulator may inhibit or alter the function of immune checkpoint proteins such as, but not limited to, lymphocyte activation gene-3 (LAG-3), T cell immunoglobulin-3 (TIM-3), T cell immunoglobulin and ITIM domain (TIGIT), V-domain Ig suppressor of T cell activation (VISTA) and B7-H3 or immune-stimulatory checkpoint pathways including but not limited to OX40, ICOS, GITR, 4-1BB and CD40 (Marin-Acevedo 2018 PMID: 29544515). Immune response modulators also include molecules targeting tumour microenvironment affected components like A2AR, HIF1α, IDO and TLR (Marin-Acevedo et al. 2018 PMID: 29544515, Naidoo *et al.* 2014 PMID: 25211661).

Examples of immune checkpoint inhibitors include, but are not limited to, anti-PD-1 pembrolizumab, nivolumab, sintilimab, camrelizumab, spartalizumab, cemiplimab, JS001 and tislelizumab, anti-PD-L1 FAZ053, LY3300054, atezolizumab and durvalumab, anti-CTLA4 AGEN1884, ipilimumab and tremelimumab, anti-LAG3 BMS986016, anti-TIM3 TSR-022, MBG453, BMS-986258 and LY3321367, anti-TIGIT MTIG7192, MK-7684, BMS-986207, OMP-313M32, AB-154 and ASP8374, anti-VISTA JNJ-61610588, anti-CD73 BMS-986179, CPI-006, and MEDI9447 and anti B7-H3 enoblituzumab.

Examples of small molecule targeted therapies include, but are not limited to, lapatinib which targets HER2 and EGFR and used in the treatment of breast cancer, neratinib which is a pan-HER family inhibitor used in the treatment of HER2+ breast cancer, anti-EGFR therapies erlotinib, gefitinib, PI3Kinase inhibitors, B-Raf inhibitors, Akt inhibitors, p53 re-activators.

Examples of receptor modulators/disruptors include but are not limited to tamoxifen and fulvestrant for the treatment of ER+ breast cancer.

Examples of immune-stimulatory therapies include but are not limited to anti-41 BB therapies (Urelumab, PF-05082566), anti-OX40 therapies (PF-04518600, MEDI6383, MEDI6469), toll-like receptor agonists (Imiquimod).

As used herein, the term "reference ADCC response" refers to an ADCC response value that can be compared with a patient-specific ADCC response to predict the patient's response to chemotherapy. Reference ADCC response value can be determined from a cohort of cancer patients in a nested study where blood samples are taken from the patients prior to chemotherapy, and the ADCC response determined from the blood samples, and the response to chemotherapy subsequently determined. The reference ADCC response is typically specific to a certain type of cancer, for example a HER2+ cancer, or a HER2+ breast cancer. Generally, the reference ADCC response is determined using the same ADCC-capable antibody, and cancer cell line, as the method employed with the patient's test sample. Generally, when the ADCC response is greater than the reference value, this is predictive of poor or non-response to chemotherapy. When the ADCC response is equal to or less than the reference value, this is generally predictive of response to chemotherapy. In one embodiment, the reference ADCC response is determined in tandem with the test sample, by employing an assay that measures ADCC response of the cell line to the ADCC-capable molecule in the presence of the blood sample but without the ICI. In this embodiment, if the ADCC response is greater in the sample containing the ICI, the non-response or poor response is predicted.

As used herein, the term "cancer patient" refers a patient in a neo-adjuvant setting (prior to surgical resection of a tumour) or in an adjuvant setting (after surgical resection), or the metastatic setting (de novo or relapse), or a patient in remission.

As used herein, the term "poor response" as applied to chemotherapy means that the patient is unlikely to have a complete response to chemotherapy. Thus, the method of the invention may be employed to identify patients in the adjuvant, metatstatic and neo-adjuvant setting that are more likely to have a partial response or non-response than a complete response to therapy.

A "non-ADCC-capable ICI" when used herein refers to an ICI that is not able to initiate an ADCC response in a patient or patient sample. Examples include but are not limited to, pembrolizumab, nivolumab, atezolizumab and durvalumab.

As used herein, the term "blood sample" or "sample of a patient's blood" refers to blood, or a fraction of blood containing effector immune cells, for example a peripheral blood mononuclear cell (PBMC) fraction, or a fraction containing specific immune cells. The cell type eliciting the antibody-dependent cytotoxic effect in this assay is PD-1 expressing and is capable of engaging of the IgG antibodies (lgG3>lgG1>lgG4>lgG2) through an Fc receptor (CD16/CD32/CD64 families) (Vidarsson et al., 2014 PMID: 25368619)

Cell types that constitutively express or can be induced to express IgG antibody-engaging receptors (CD16a/b, CD32a/b/c, CD64) include NK cells, T cell subsets, B cells, dendritic cells (DCs), monocytes, macrophages, neutrophils, eosinophils, basophils and mast cells (Bruhns et al., 2012 PMID: 22535666, DiLillo and Ravetch 2015 PMID: 26138698.2015).

Cell types that are potentially present as effector cells in our assay include lymphocytes (NK cells, B cells, T cells, dendritic cell subtypes), monocytes/macrophages and granulocytes (neutrophils, eosinophils, basophils and mast cells) (Amersham Biosciences Handbook Ficoll-Paque Plus 18-1152-69 Edition AB).

HER2+ or HER2 overexpressing cancers are well known in the art and it will be appreciated that any such cancer can apply (Wolff et al., 2018 PMID: 29846104). HER2 low or HER2 negligible or HER2 negative cancers are well known in the art and any such cancer can apply. HER2 expression levels are best defined in HER2+ breast cancer with variations on the methodology applied to other cancers e.g. gastric cancer (Lordick et al. 2017 PMID: 28285403) and ovarian cancer (Tuefferd et al. 2007 PMID: 17987122). The HER2 status of cell lines is assessed in a similar manner to the clinical setting whereby HER2 copy number and protein expression levels provide the basis for a HER2+/HER2 over-expressing classification versus a HER2-low or HER2 negligible or HER2 negative classification.

The phrase "immune cell mediated cytotoxicity" when used herein refers to the killing of cells, or cancer cells, by immune cells by any method they are capable of killing including but not limited to ADCC, ADCP and direct cytotoxicity.

The phrase "primary cell line" when used herein refers to normal calls or cancer cells taken directly from a patient or a healthy volunteer that is not immortalised.

The phrase "direct cytotoxicity" when used herein refers to an immune cell directly killing a cell, or cancer cell, in the absence of an intermediary, such as, but not limited to, an ADCC - capable molecule.

### Brief Description of the Figures

The invention will be more clearly understood from the following description of an embodiment thereof, given by way of example only, with reference to the accompanying drawings, in which:

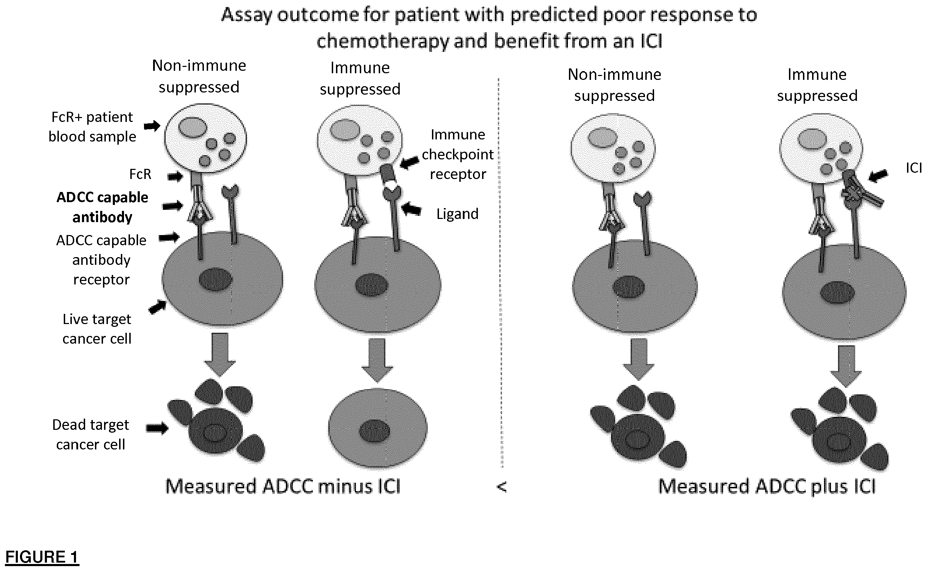

**Figure 1****:** Graphic displaying the underlying basis of the IP. A blood sample from the patient is divided in two and assayed in the presence of an ADCC-capable molecule (e.g. an ADCC capable antibody) and a target cell line expressing the target of the ADCC-capable antibody (left side of figure). The other part of the blood sample is assayed in the same way but in the presence of an immune response modulator e.g. an immune checkpoint inhibitor (right side of figure). If the ADCC response (as measured by the number of target cancer cells killed in the assay) is greater in the presence of the ICI, then that patient is predicted to have a poor response to chemotherapy and gain benefit from ICI therapy. Should the level of ADCC measured be the same under the two conditions tested, these patients will have an increased chance of a better response to chemotherapy. FcR = FC receptor. ICI = immune checkpoint inhibitor.

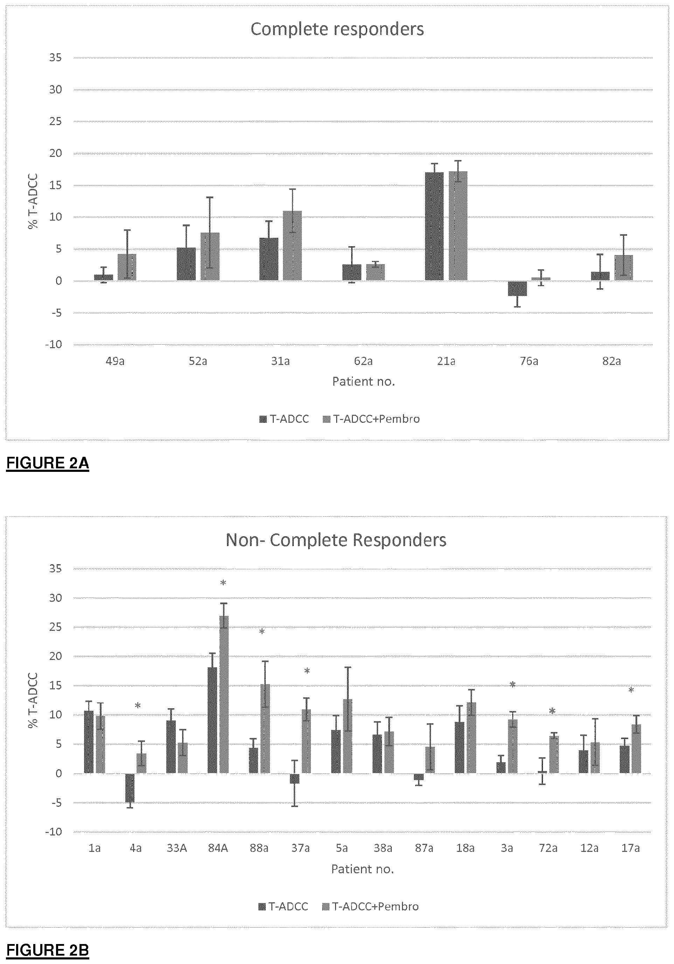

**Figure 2****:** Levels of trastuzumab-mediated ADCC (T-ADCC) against the HER2+ SKBR3 breast cancer cell line with (test) and without (reference) the anti-PD-1 immune checkpoint inhibitor pembrolizumab in complete responders (CR - Fig. 2A) and non-complete responders (nCR - Fig. 2B). 0/7 CR had significant increase in T-ADCC with the addition of pembrolizumab. 7/14 nCR had a significant increase in T-ADCC with the addition of pembrolizumab. Significance denoted by *. Significance determined by Student's T test with * = p<0.05.

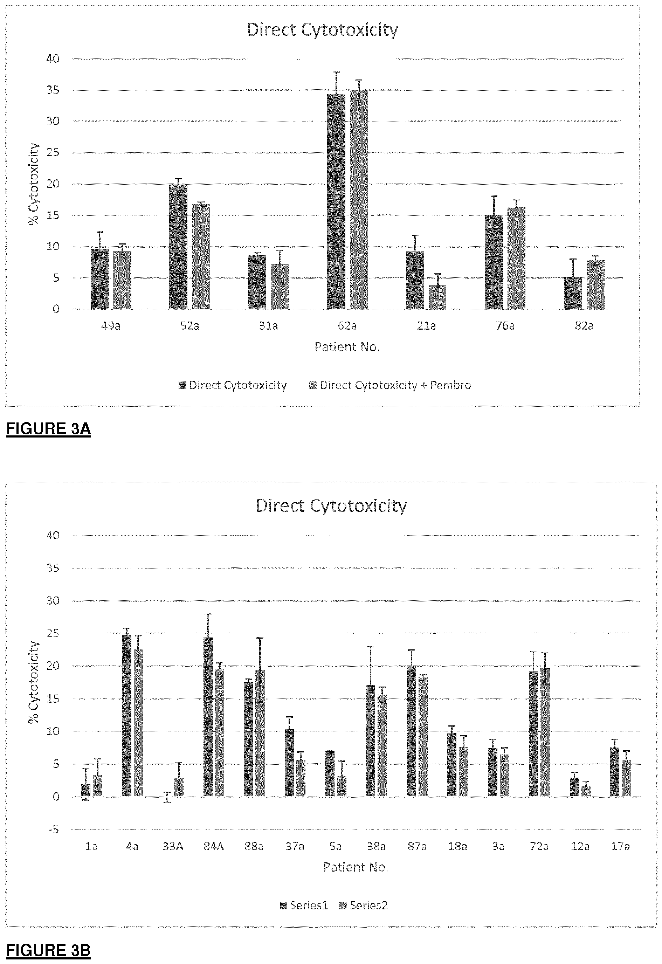

**Figure 3****:** Levels of direct cytotoxicity against the leukemic cell line K562 with (test) and without (reference) anti-PD-1 immune checkpoint inhibitor pembrolizumab in complete responders (CR - Fig. 3A) and non-complete responders (nCR - Fig. 3B). K562 cells are non-MHC-restricted and are therefore particularly sensitive to direct immune cell killing by NK cells. There was no significant change in direct cytotoxicity *in vitro* for any patient with the addition of pembrolizumab suggesting the effect is ADCC-related. Significant increase in cytotoxicity denoted by *. Significance determined by Student's T test with * = p<0.05.

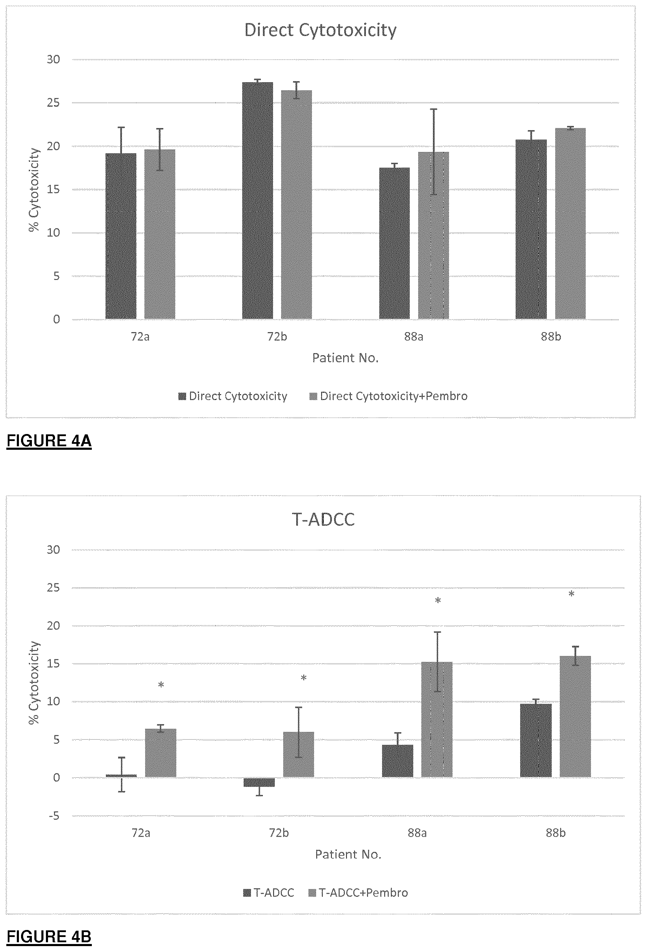

**Figure 4****:** Effect of anti-PD-1 immune checkpoint inhibitor pembrolizumab on T-ADCC against the SKBR3 cell line is maintained following 6 cycles of chemotherapy (~ 4 months) in nCR patients 72 and 88. Fig. 4A). Levels of direct cytotoxicity with (test) and without (reference) pembrolizumab in pre- and post-treatment samples for patient's no. 72 and 88. Fig. 4B) Levels of T-ADCC with (test) and without (reference) pembrolizumab in pre-and post-treatment samples for patient's no. 72 and 88. Fig 4B suggests that the effect being measured in the assay is stable over a relatively long period of time (~4 months) and could be utilised before, during or after treatment to determine the suppressive state of a patient's immune cells. Significant increase in cytotoxicity denoted by *. Significance determined by Student's T test with * = p<0.05.

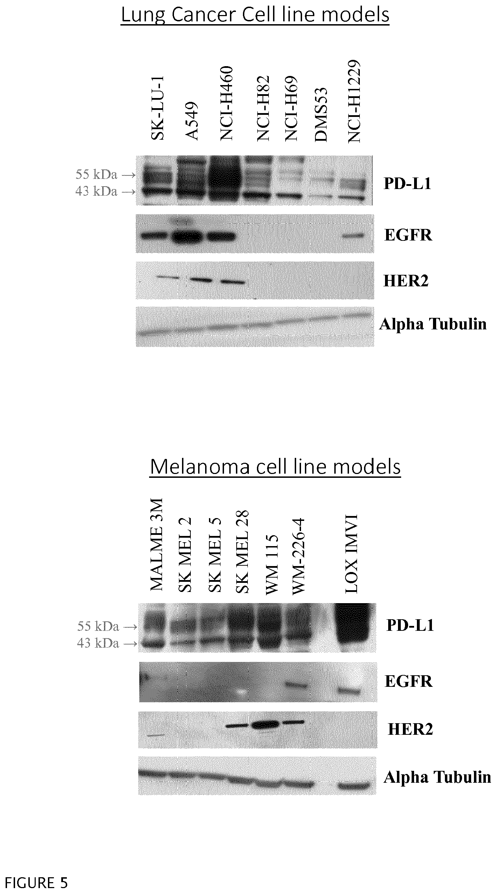

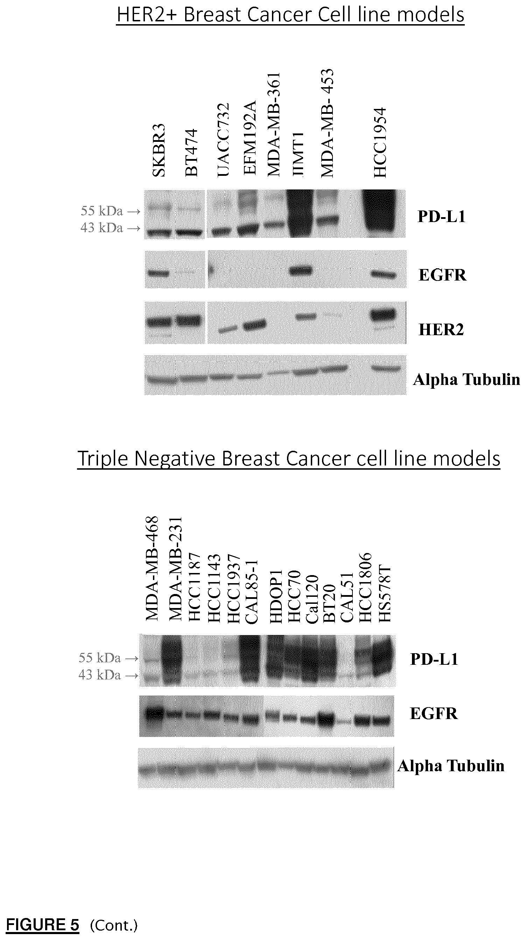

**Figure 5****:** Expression of immune checkpoint protein PD-L1 and targets for therapeutic ADCC capable antibody therapies EGFR and HER2 in panels of lung (n=7), melanoma (n=7) and breast cancer (HER2+ (n=8)/triple negative(n=13)) cell lines by Western blot. 30 µg of protein was examined for all cell lines. Alpha-tubulin was used as a loading control. PD-L1 appears as a multiple band due to various glycosylation states. This figure shows the availability of cell lines within cancer types that express EGFR or HER2 meaning they can be used as target cells in our method using cetuximab or trastuzumab, respectively, as the ADCC-capable antibody. Knowledge of the PD-L1 expression levels in a cell line is also a key parameter when choosing a target cell line. PD-L1 -low cell lines, for example, may be preferable when utilising an anti-PD-1 ICI to eliminate the impact of PD-1/PD-L1 interactions on antibody action.

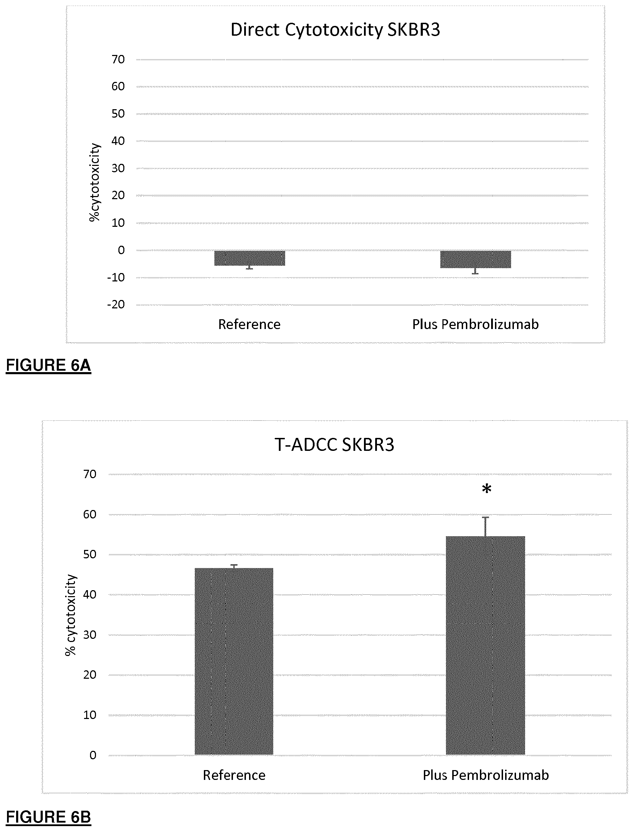

**Figure 6****:** A) Direct cytotoxicity elicited by PBMCs from a blood sample from metastatic lung cancer patient against the HER2+ SKBR3 breast cancer cell line with (test) and without (reference) pembrolizumab treatment B) T-ADCC elicited by PBMCs from the same blood sample against the SKBR3 breast cancer cell line with (test) and without (reference) pembrolizumab treatment. Significant increase in cytotoxicity denoted by *. Significance determined by Student's T test with * = p<0.05.

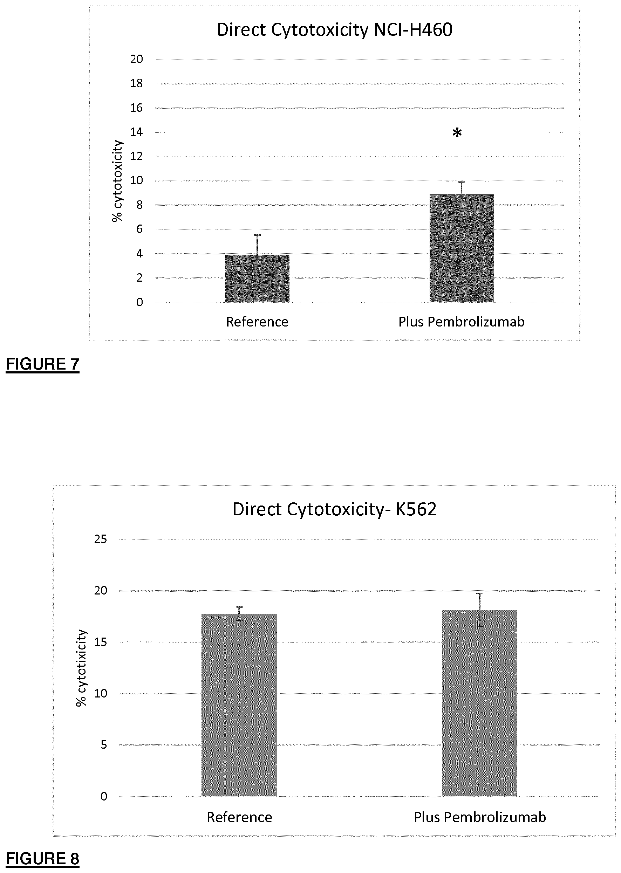

**Figure 7****:** Direct cytotoxicity elicited by PBMCs from a blood sample from a metastatic lung cancer patient against HER2-low NCI-H460 lung cancer cell line with (test) and without (reference) pembrolizumab treatment. Significant increase in cytotoxicity denoted by *. Significance determined by Student's T test with * = p<0.05.
**Figure 8****:** Direct cytotoxicity elicited by a blood sample from a metastatic lung cancer patient against the K562 leukemic cell line with (test) and without (reference) pembrolizumab treatment.

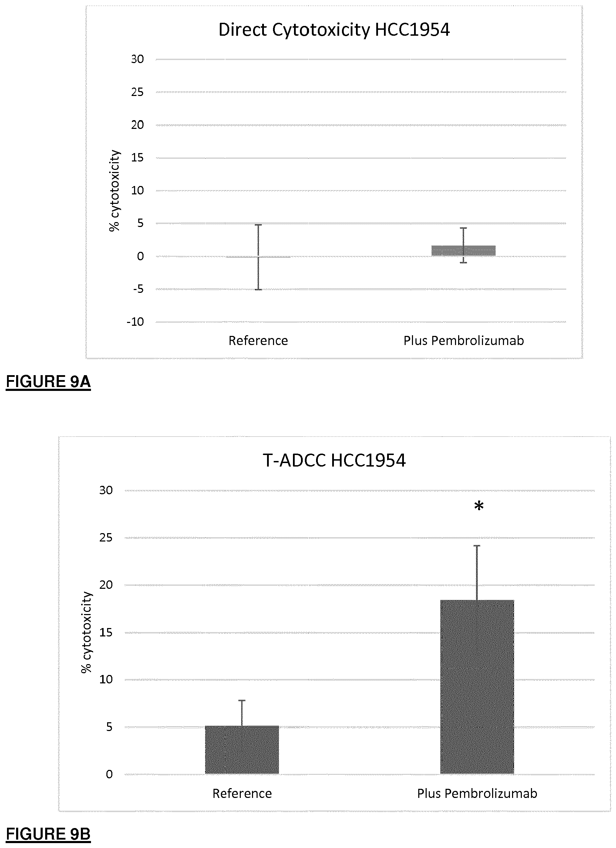

**Figure 9****:** (A) Direct cytotoxicity elicited by PBMCs from a blood sample from a melanoma patient against the HER2+ HCC1954 breast cancer cell line with (test) and without (reference) pembrolizumab treatment; (B) T-ADCC elicited by PBMCs from a blood sample from the same patient against the HCC1954 breast cancer cell line with (test) and without (reference) pembrolizumab treatment. Significant increase in cytotoxicity denoted by *. Significance determined by Student's T test with * = p<0.05.

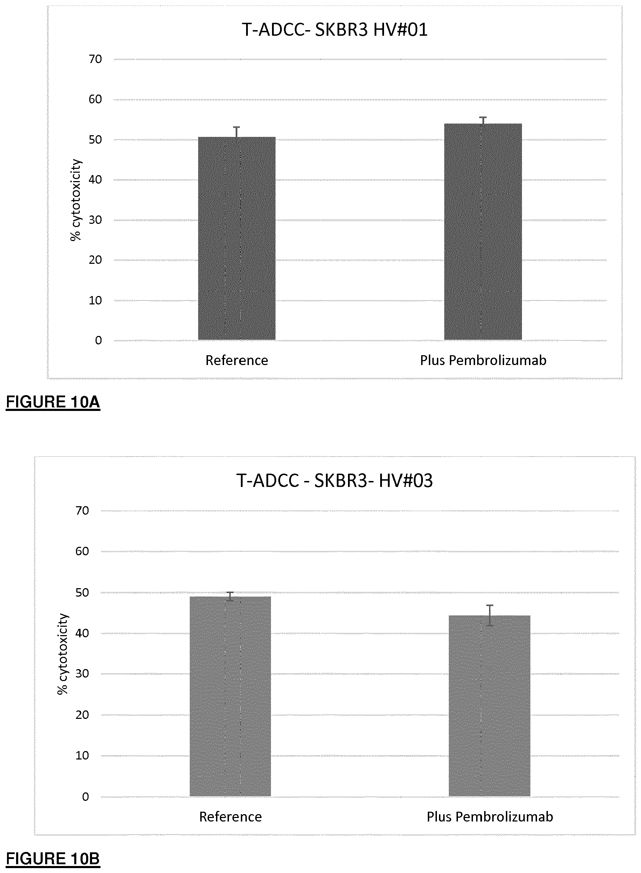

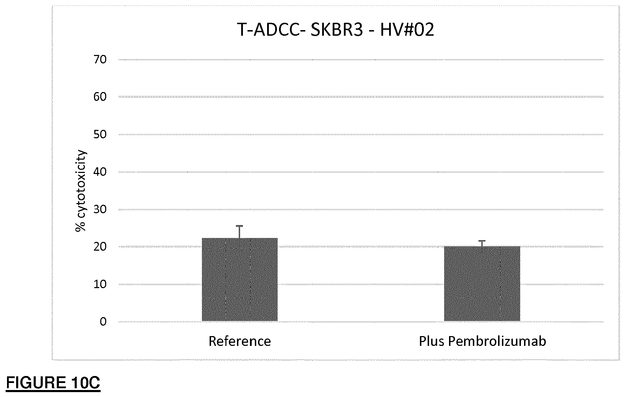

**Figure 10****:** T-ADCC elicited by PBMCs isolated from blood samples from 3 separate healthy volunteers (Figure 10 A to C) against the HER2+ SKBR3 breast cancer cell line with (test) and without (reference) pembrolizumab treatment. No significant increase in cytotoxicity was detected in the presence of pembrolizumab.

### Detailed Description of the invention

There is a current need to identify patients who would benefit from particular cancer treatment methods and those who will not. If the method indicates a non-response or a likelihood of a poor response, this will increase the likelihood of surgery while avoiding putting a patient through a treatment regimen that is unlikely to work while having severe side effects. In this sense, the method of the invention fast tracks a patient to the most effective treatment strategy.

The current inventors have surprisingly discovered that measuring the ADCC response to trastuzumab (Herceptin) in the presence of an ICI using a patient's blood sample may be used to predict the likelihood of response to a cancer treatment strategy. The results shown by the inventors are surprising as the accepted wisdom is that ICls have no effect on the ADCC response in a laboratory setting.

The method of the invention works by identifying a patient group who have a suppressed anti-tumour immune response or "exhausted" immune cells circulating in the blood.

The current invention provides an *in vitro* method for predicting or determining an individual's response to a treatment for cancer as per the appended claims. The method comprises providing a blood sample from a patient. It will be appreciated that the blood sample may be taken from a patient at any time before treatment starts, at any time during treatment and/or after treatment is finished. The blood sample is preferably isolated from the patient before any type of cancer treatment commences, or during cancer treatment, or after an initial cancer treatment and prior to a subsequent treatment.

It is preferred that the blood sample is an immune cell subset or mixed immune cell population, such as the PBMC fraction, which contains functionally cytotoxic cells. The immune cell population may be isolated from a whole blood sample by methods known in the art. It will be appreciated that any such method may be used. Preferably, the fraction, such as the PBMC fraction, is isolated by density centrifugation or by magnetic bead separation techniques, cell preparation tube (CPT) isolation techniques, isolation using SepMate ^{™} tubes or by any suitable technique.

The mixed immune cell population, such as the PBMC fraction, may be isolated at any time after blood draw, preferably between 0 and 4 hours, typically within four hours of blood draw from the patient. Preferably, the cells, e.g. PBMC, are counted and such methods are known in the art. The cells, e.g. PBMC, may be checked for viability and, again, such methods are known in the art and any such method may be used.

The blood sample may be divided into at least two fractions, typically a first fraction and a second fraction.

The method involves a step of adding an amount of the ADCC capable molecule, such as, trastuzumab, to the second fraction of the blood sample.

The method involves a step of adding an amount of the ADCC capable molecule, such as trastuzumab, and an amount of an immune checkpoint protein modulator, such as an ICI, are added to the first fraction of the blood sample.

The ICI may be any ICI known in the art. Preferably, the ICI may be a non-ADCC-capable ICI. The ICI may be selected from, but not limited to, the group comprising pembrolizumab, nivolumab, atezolizumab and durvalumab. The ICI may be avelumab or ipilimumab which are ICls that engage in ADCC.

The first fraction is added to a first cancer cell line. The second fraction is added to a separate second cancer cell line. The first and second cancer cell line may be the same or it may be different. It will be understood that any suitable cancer cell line may be used. Preferably, the cancer cell lines is a breast cancer cell line. Typically, the cell line is a HER2+ breast cancer cell line, for example, SKBR3. The cell line may be a matched cell line. The cell line may be an unmatched cell line.

The cancer cell line used expresses, preferably high levels of expression, a target of the ADCC capable molecule. This target may be an antigen target. The cancer cell line may be susceptible to ADCC. The cancer cell line may have low, negligible or no expression of the target of the ICI used in the method of the invention.

The ADCC response of the first fraction against the first cell line is measured. The ADCC response of the second fraction against the second cell line is measured. Methods of measuring an ADCC response are known in the art and it will be appreciated that any such method may be used. Preferably, the ADCC response is measured by a flow cytometry based ADCC assay.

The ADCC response is essentially a measure of the percentage of dead cells attributable to the agent added to the cancer cell line, i.e. the trastuzumab alone, or trastuzumab and ICI.

For example, ADCC may be calculated by subtracting (determining the difference) the amount of cells killed by immune cells without ADCC-capable molecule, e.g. trastuzumab from the amount of cells killed with ADCC-capable molecule, e.g. trastuzumab. T-ADCC in the presence of an ICI is calculated by subtracting the amount of cells killed by immune cells with the ICI (without trastuzumab) from the amount of cells killed by immune cells with the ICI and the ADCC capable molecule, e.g. trastuzumab.

Methods of measuring an ADCC response are known in the art and it will be appreciated that any such method may be used. Preferably, the ADCC response is measured by a flow cytometry based ADCC assay. Other ADCC assay systems include LDH-based assays (enzymatic-based), chromium release assays (radioactivity-based), calcein-AM assays (fluorescence-based), high content analysis-based assays (image-based), Xcelligence^{®}-based (impedance-based) assays.

In the current method, the ADCC response of the first fraction is compared with the ADCC response of the second fraction.

Detection of a higher ADCC response in the first fraction compared to the ADCC response in the second fraction is predictive of a poor response to said treatment. Accordingly, this result indicates that the patient is unlikely to have a good outcome in response to said treatment and alternative treatment strategies, such as ICI treatment or surgery, should be considered.

The incubation of the method of the invention is for a period of time. It will be appreciated that any suitable period of time may be used sufficient to provide an immune cell mediated cytotoxicity indication, e.g. an ADCC response. It is known to the person skilled in the art. For example, the period of time may be from 30 seconds to 48 hours, from 1 minute to 24 hours, from 5 minutes to 12 hours, from 10 minutes to 10 hours, from 4 hours to 6 hours, it may be 30 minutes, 1 hour, 2 hours, 3 hours, 4 hours, 5 hours, 6 hours, 7 hours, 8 hours or 9 hours.

Not wishing to be bound by theory, the inventors believe that trastuzumab in the first and second fraction binds to HER2 on the cancer cell line. An ADCC-capable immune cell in the patient sample then binds to the trastuzumab and kills the tumour cell unless it is suppressed through immune checkpoint protein expression, e.g. PD-1 expression. The ICI binds to the immune checkpoint protein on the ADCC-capable immune cell, releases the suppression and allows the ADCC-capable immune cell to kill the tumour cell through the engagement with bound trastuzumab. In this way, the method of the invention is detecting a specific cell phenotype, i.e. a patient with immune cells that are suppressed and thus ones that are likely to not produce an immune response in the event of antibody therapy or chemotherapy treatment unless the suppression is removed with an ICI. As such, the method of the invention has the capacity to identify cell types in the patient that are functionally inhibited and therefore, any immune therapy that acts by increasing immune cell activity could be tested, e.g. immune checkpoint inhibitors (anti-PD1/anti-PDL1/anti-CTLA4), co-stimulatory molecules (anti-41 BB, anti-OX40), anti IDO therapies, anti A2AR therapies and anti CD73 therapies.

Typically, the method of the invention may be used to test patients who are suffering from any cancer. Circulation of immune-suppressed, ADCC-capable immune cells could potentially occur in any cancer due to the production of autologous anti-tumour antibodies by the patient's bodies. Preferably, the cancer is also eligible for treatment with an immune stimulating cancer therapy e.g. ADCC-capable monoclonal antibody therapy (eg trastuzumab, pertuzumab, cetuximab, margetuximab) or classical chemotherapies such as docetaxel, paclitaxel etc. Alternatively, the cancer is selected from, but not limited to, the group comprising melanoma, lung cancer, gastric cancer, bladder and ovarian cancer. The breast cancer may be HER2+, ER-positive or triple negative (TN)-breast cancer.

The cancer treatment referred to in the method of the invention may be breast cancer treatment, preferably a HER2+ breast cancer treatment. The cancer treatment may be docetaxel treatment alone. The cancer treatment may be carboplatin treatment alone. Alternatively, the treatment may be a combination treatment in which the patient receives both docetaxel and carboplatin. In an embodiment, the treatment is trastuzumab treatment. In one embodiment, the treatment may be a combination treatment in which the patient receives docetaxel, carboplatin and trastuzumab. Other trastuzumab containing treatment regimens for HER2+ breast cancer: ACTH consists of Adriamycin, cyclophosphamide, paclitaxel. TH consists of paclitaxel (sister drug to docetaxel) and trastuzumab. THP consists of docetaxel, trastuzumab and pertuzumab. The cancer may be any cancer, e.g. HER2 negative. The cancer may be lung cancer.

In an embodiment, the cancer treatment may be an ICI treatment. The ICI given to the patient may be any suitable ICI useful for the treatment of the cancer type in question. The ICI may be as described herein.

In an embodiment, the treatment for cancer is cetuximab. It will be understood that the treatment is determined by the cancer type that the patient is suffering from and such treatments are known in the art.

The method may involve determining direct cytotoxicity as a means to predict response to chemotherapy, or an immune response modulator, in a patient. The blood sample of the patient may be separated into two fractions. The method involves a step of adding an amount of an immune response modulator, such as an ICI, to a first fraction of the blood sample.

The first fraction is added to a first cancer cell line. The second fraction is added to a separate second cancer cell line. The first and second cancer cell line may be the same or it may be different. It will be understood that any suitable cancer cell line may be used. Preferably, the cancer cell line is a breast cancer cell line. Typically, the cell line is a HER2 negative cancer or HER2 low cancer cell line. The cell lines may be HER2+ cell line.

The direct cytotoxicity of the first fraction against the first cell line is measured. The direct cytotoxicity of the second fraction against the second cell line is measured. Methods of measuring direct cytotoxicity are known in the art and it will be appreciated that any such method may be used.

Detection of a higher direct cytotoxicity in the first fraction compared to that of the second fraction is predictive of a poor response to chemotherapy. Detection of a higher direct cytotoxicity in the first fraction compared to that of the second fraction may be predictive of a good response to a treatment using an immune response modulator. Embodiments as disclosed herein with reference to any method of the invention, are also applicable to this method of the invention.

### EXAMPLES

### Example 1

### Exemplary Assay setup

PBMCs were isolated from patients' whole blood using a standard Ficoll-Paque-based centrifugation protocol. Isolated PBMCs were then frozen in cryovials at a concentration of 1 × 10⁷ cells/ml. in freezing solution (Heat-inactivated foetal bovine serum (FBS) + 5% DMSO). The cryovials were frozen at -80°C in Mr. Frosty units and transferred to liquid nitrogen for long term storage. PBMCs could also be used directly in assays following isolation.

PBMCs (effector cells - ECs) were revived by thawing the cryovials in a 37° water bath. Cells were then transferred to sterile tubes, 10ml of warmed assay medium (filter sterilised RPMI1640, 10% heat-inactivated FBS and 0.5% pen/strep) was added. The tubes were centrifuged and re-suspended in 5 ml. warm assay media. Cell concentration and viability was determined using the Guava viacount method.(http://www.merckmillipore.com/IE/en/product/Guava-ViaCount-Reagent-for-Flow-Cytometry-100-tests,MM NF-4000-0040#documentation. The revived PBMCs were incubated at 37°C for 4-5 hours before starting the ADCC assay.

Target cells (TCs) were grown to 70-80% confluency for use in the assay. TCs were washed twice with PBS and incubated with carboxyfluorescein succinimidyl ester (CFSE) in PBS. Following a 20 min incubation, CFSE was removed from the TCs which were then washed twice with PBS and incubated in 20 ml assay medium to quench excess CFSE. Adherent TCs were trypsinised and centrifuged. The pellet was washed twice in PBS and re-suspended in assay medium. Non-adherent TCs were centrifuged, washed twice in PBS and re-suspended in assay medium. Cell viability was determined using the Guava viacount method. Target cells and PBMCs were then brought to the same concentration, 1.0 - 2.5 × 10⁵ cells/ml. Final concentration used was dependent on cell count and viability levels. TCs and ECs were then divided and treated as control (no treatment) or with trastuzumab (10 µg/ml), pembrolizumab (10 µg/ml) and trastuzumab and pembrolizumab (10 µg/ml). Control wells included basal cell death, 100% dead cell controls (TCDCC), PBMCs only and a negative control for ADCC (CD20-specific rituximab). Cells were plated as shown in Table 1. The plates were centrifuged at 50 g for 3 minutes and incubated for 4 hours (or 12 hours). 5 µl of Triton-X was then added to the dead cell control wells and 40 µl of 35 µM 7-aminoactinomycin-D (7AAD) was added to all wells to stop the assay by gently re-suspending the cells. The plates were then read on a Guava Easycyte flow cytometer (Millipore) within 6 hours using the Guava InCyte program to determine the viability of the target cells in each condition.

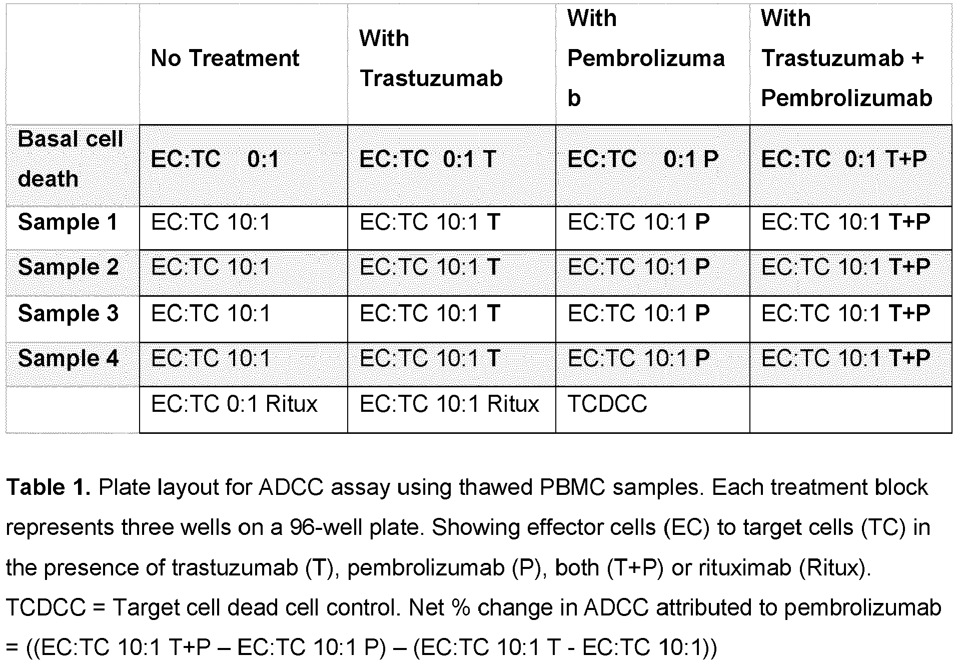

### Example 2

### ADCC response against HER2+ cell line SKBR3 in the presence of trastuzumab and in the present of trastuzumab and the ICI pembrolizumab.

Blood samples were taken from 21 patients before receiving cancer treatment. After the samples were taken all 21 patients received the cancer treatment. All patient's received docetaxel and carboplatin (chemotherapy backbone), along with trastuzumab alone (n=5) or trastuzumab with lapatinib (n=14) or lapatinib alone (n=2). The cancer treatment administered was docetaxel and carboplatin along with trastuzumab (Herceptin). A mixed population of immune cells from the patients called peripheral blood mononuclear cells (PBMCs) were isolated from the patient's whole blood samples. PBMCs were separated from the blood sample taken using a standard Ficoll-Paque-based centrifugation assay (Brayum, 1968, PMID: 4179068)/Bøyum 1968, PMID: 4179066).

Cancer cells were placed in a first dish with PBMCs and an amount of trastuzumab and a second dish with PMBCs and an amount of trastuzumab and an amount of pembrolizumab.

The amount of breast cancer cells killed by PBMCs was measured.

The amount of breast cancer cells that were killed due to the ADCC response to trastuzumab alone and in the presence of an ICI was measured using a flow cytometry-based method. The patients were separated into their known response to chemotherapy, i.e. whether they were non-complete responders (nCR) or complete-responders (CR).

### Results

7 of the 21 patients tested showed an increased ADCC response to trastuzumab in the presence of the ICI and all 7 of these patients were non-complete responders to chemotherapy (Figure 2B). 0/7 complete responders exhibited increased ADCC response to trastuzumab in the presence of the ICI (Figure 2B).

The amount of K562 cells (non-MHC restricted, sensitive to direct effector cell killing) killed by PBMCs was measured. The results are shown in Figure 3A (complete responders - CR) and Figure 3B (non-complete responders - nCR). Direct killing of cancer cells by PBMCs is postulated to be mainly mediated by NK cells. The presence of pembrolizumab did not alter the direct killing of cancer cells by PBMCs (Figure 3A and 3B).

### Conclusion

The impact of the ICI pembrolizumab on the ADCC response to trastuzumab is a potential biomarker of response to chemotherapy (Figure 2A and 2B - 7/14 (50%) non-complete responders (PR/NR), 0/7 (0%) complete responders (CR)).

### Later treatment

Of the 7 patient's that showed a response to the ICI pembrolizumab *in vitro,* i.e. an increased ADCC response, one did not receive trastuzumab. Therefore, it will be appreciated that the method of the invention could be a means to predict response to cytotoxic chemotherapy backbones e.g. docetaxel and carboplatin, and other classical chemotherapeutics (paclitaxel, cisplatin, anthracyclines), and not only targeted therapies like trastuzumab.

Classical chemotherapeutics like docetaxel and carboplatin alone could activate the anti-cancer immune response under the right conditions through the killing of tumour cells and release of tumour cell content, the killing of pro-cancer immune cells, and enhancement of the anti-tumour effects of CD8+ T cells, pushing the patient's' normal immune response to take over and produce their own anti-tumour antibodies (not trastuzumab) (Garnett et al., 2008, PMID: 18519787). This mechanism could be employed in the patients that have a complete response to chemotherapy as they do not have PD-1 suppressed immune cells. In this manner, it is considered that the method is detecting patients with suppressed immune cells suggesting that there is an anti-cancer immune response mounted in the body that needs intervention of an ICI chemotherapy to produce a complete response to chemotherapy. For the 50% of non-complete responders that do not show a response to the ICI in the method, it is suggested that their poor response to chemotherapy is down to factors other than PD-1-related immune suppression.

### Example 3

### Choice of target cell line for assay

### Materials and Methods

EGFR and HER2 levels in 30 µg of cell lysate were measured in lung (n=7), melanoma (n=7) and breast cancer (HER2+ (n=8) and triple negative (=13)) cell lines by Western blot. PD-L1 expression in the same panels of cell lines was also determined by Western Blot. Alpha-tubulin was utilised as a loading control.

### Results

The results suggest that multiple cell lines could be utilised for assays specific to a cancer type utilising, for example, EGFR and HER2 as targets for the ADCC-capable antibodies cetuximab and trastuzumab, respectively (Figure 5). PD-L1 expression levels vary greatly between cell lines within cancer types. In general, the levels of the target of the immune checkpoint inhibitor being used and the cognate ligand of the receptor should be assessed on the target cell line to ensure that the assay will perform correctly (Figure 5).

### Example 4

### Method of Calculation

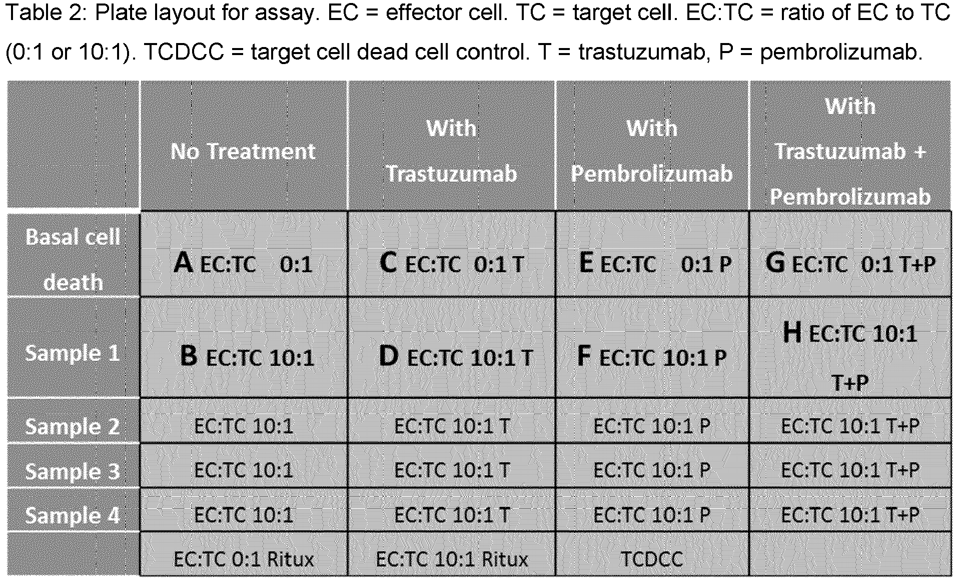

Technical triplicate results for the % dead target cells for each treatment is obtained as in the plate layout above. Each grey box represents three wells on a 96 well plate. 4 samples were run on the above plate, with rituximab control (negative control for antibody-dependent cytotoxicity) and dead target cell controls. The following denotes the calculations for Sample 1, which were repeated for Samples 2, 3 and 4. For simplicity, the basal cell death and Sample 1 treatments have been labelled A to H to follow the calculations.

To obtain a value for direct cytotoxicity: an average of the three values for "A" is subtracted from each individual value for "B". These three values are then averaged and the standard deviation for the triplicate values is calculated.

The same is done for conditions "E and F" to calculate direct cytotoxicity while pembrolizumab is present.

To obtain a value for T-ADCC: An average of the three values for "B" is subtracted from each individual value for "D". These three values are then averaged and the standard deviation for the triplicate values is calculated.

The same is done to calculate T-ADCC with pembrolizumab: An average of the three values for "F" is subtracted from each individual value for "H". These three values are then averaged and the standard deviation for the triplicate values is calculated.

### Example 5

### ADCC response and direct cytotoxicity by PBMCs from a lung cancer patient.

### Materials and Methods

Blood sample was taken from a patient with metastatic lung cancer before receiving cancer treatment. After the sample was taken the patient received pembrolizumab, an anti-PD-1 antibody.

A mixed population of immune cells from the patients called peripheral blood mononuclear cells (PBMCs) were isolated from the patient's whole blood samples. PBMCs were separated from the blood sample taken using a standard Ficoll-Paque-based centrifugation assay (Brayum, 1968, PMID: 4179068)/Bøyum 1968, PMID: 4179066).

The following cancer cell lines were used:
HER2+ SKBR3 breast cancer cell line.
HER2-low NCI-H460 lung cancer cell line.
K562 leukemic cell line.

The following was carried out for SKBR3.

Cancer cells were placed in a first dish with PBMCs and an amount of trastuzumab and a second dish with PMBCs and an amount of trastuzumab and an amount of pembrolizumab. The amount of cancer cells killed by PBMCs was measured.

The amount of cancer cells that were killed due to the ADCC response to trastuzumab alone and in the presence of an ICI was measured using a flow cytometry-based method.

The following was carried out for NCI-H460 and K562.

Cancer cells were placed in a first dish with PBMCs and a second dish with PMBCs and an amount of pembrolizumab. The amount of cancer cells killed by direct cytotoxicity by PBMCs was measured in both using a flow cytometry-based method.

### Results

### SKBR3

The patient showed an increased ADCC response to trastuzumab in the presence of the ICI (Figure 6B) against SKBR3 breast cancer cell line. Direct cytotoxicity elicited by PBMCS was measured. The results are shown in Figure 6A. Direct killing of cancer cells by PBMCs is postulated to be mainly mediated by NK cells. The presence of pembrolizumab did not alter the direct killing of cancer cells by PBMCs (Figure 6A). SKBR3 has negligible levels of PD-L1.

### NCI-H460

Direct cytotoxicity elicited by PBMCs was measured. The results are shown in Figure 7. The presence of pembrolizumab did alter the direct killing of cancer cells by PBMCs.

### K562

Direct cytotoxicity elicited by a blood sample from a metastatic lung cancer patient against the K562 leukemic cell line with and without pembrolizumab treatment was measured and the results are illustrated in Figure 8. There was no change in cytotoxicity in the presence of pembrolizumab. The K562 cell line is used as a benchmark to ensure that there is not a sample with an unusually high or low cytotoxic activity.

### Conclusion

As the results of Figure 6B are in line with those of Figure 2, it is plausible that the ADCC response in the presence of an ICI is a potential biomarker of response to chemotherapy for non-breast HER2+ cancers, such as lung cancer.

The effect of the ICI pembrolizumab on the ADCC response to trastuzumab is present in non-HER2+ breast cancer patient blood samples and could be a potential biomarker of response to chemotherapy for non-HER2+ cancer patients. The presence of an ICI responsive, ADCC-capable immune cell in the blood of this lung cancer patient suggests the presence of the same cell type found in the HER2+ breast cancer patients who did not respond to chemotherapy and therefore that this patient may have a poor response to chemotherapy.

It is plausible that the presence of PD-1 suppressed functional immune cells may indicate a beneficial response to a PD-1 targeted therapy.

The same cytotoxic cell population which are capable of eliciting increased levels of ADCC may also be capable of increasing direct cytotoxicity when PD-1-mediated immunosuppression is relieved. This may indicate a role for alterations to direct cytotoxicity, which is measured as part of the assay described, in predicting response to chemotherapy.

### Example 6

### ADCC Response by PBMCs from a melanoma patient against cell lines in the presence of trastuzumab and in the presence of trastuzumab and the ICI pembrolizumab

Blood sample was taken from a patient with melanoma before receiving cancer treatment. After the sample was taken the patient received pembrolizumab, an anti-PD-1 antibody.

A mixed population of immune cells from the patients called peripheral blood mononuclear cells (PBMCs) were isolated from the patient's whole blood samples. PBMCs were separated from the blood sample taken using a standard Ficoll-Paque-based centrifugation assay (Brayum, 1968, PMID: 4179068)/Bøyum 1968, PMID: 4179066).

The following cancer cell lines were used:
HER2+ HCC 1954 breast cancer cell line.

Cancer cells were placed in a first dish with PBMCs and an amount of trastuzumab and a second dish with PMBCs and an amount of trastuzumab and an amount of pembrolizumab. The amount of cancer cells killed by PBMCs was measured.

The amount of cancer cells that were killed due to the ADCC response to trastuzumab alone and in the presence of an ICI was measured using a flow cytometry-based method.

### Results

Direct cytotoxicity elicited by PBMCs from a blood sample from a melanoma patient against the HER2+ HCC1954 breast cancer cell line with and without pembrolizumab treatment is illustrated in Figure 9(A). Figure 9 (B) shows trastuzumab-mediated ADCC elicited by PBMCs from a blood sample from the same patient against the HCC1954 breast cancer cell line with and without pembrolizumab treatment * denotes p value of <0.05

### Conclusion

An increase in T-ADCC with addition of pembrolizumab was seen in the test sample. It is plausible that the ADCC response in the presence of an ICI is a potential biomarker of response to chemotherapy.

### Example 7

### ADCC response using samples from healthy volunteers

### Materials and Methods

Blood samples were taken from 3 separate healthy volunteers.

A mixed population of immune cells from the patients called peripheral blood mononuclear cells (PBMCs) were isolated from the healthy volunteers' whole blood samples. PBMCs were separated from the blood sample taken using a standard Ficoll-Paque-based centrifugation assay (Brayum, 1968, PMID: 4179068/Bøyum 1968, PMID: 4179066).

HER2+ SKBR3 breast cancer cell line was used.

Cancer cells were placed in a first dish with PBMCs and an amount of trastuzumab and a second dish with PMBCs and an amount of trastuzumab and an amount of pembrolizumab. The amount of cancer cells killed by PBMCs was measured. The amount of cancer cells that were killed due to the ADCC response to trastuzumab alone and in the presence of an ICI was measured using a flow cytometry-based method.

### Results

No significant increase in trastuzumab-mediated ADCC with pembrolizumab when healthy volunteer immune cells were used as the effector cell population (Figure 10 A to C).

### Conclusion

The test response is specific to cancer patients.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A method of predicting response to chemotherapy in a patient with cancer, the method comprising the steps of:
incubating an ADCC-capable molecule, an immune checkpoint protein modulator, a cancer cell line that expresses a target of the ADCC-capable molecule, and a sample of the patient's blood, for a period of time;
determining an ADCC response against the cancer cell line in the presence of said immune checkpoint protein modulator;
comparing the ADCC response with a reference ADCC response in the absence of an immune checkpoint protein modulator; and
predicting the patient's response to chemotherapy based on the comparison,
wherein an ADCC-capable molecule refers to a molecule that is capable of binding to a cell surface protein, or antigen, such as a receptor, on the tumour surface, and generating an antibody-dependent cell-mediated cytotoxicity (ADCC), or antibody-dependent cell mediated phagocytosis (ADCP) response, against the tumour cell whereby immune cells bind the molecule and kill the tumour cell.

2. A method according to Claim 1, in which the immune checkpoint protein modulator is an immune checkpoint inhibitor (ICI) or an immune checkpoint protein stimulator.

3. A method according to Claim 2, in which the immune checkpoint protein modulator is an ICI, preferably in which the ICI is selected from pembrolizumab, nivolumab, atezolizumab and durvalumab, avelumab and ipilimumab.

4. The method according to any one of the preceding claims, in which the checkpoint protein is selected from the group comprising lymphocyte activation gene-3 (LAG-3), T cell 5 immunoglobulin-3 (TIM-3), T cell immunoglobulin, ITIM domain (TIGIT), V-domain Ig suppressor of T cell activation (VISTA), B7-H3, an immune stimulatory checkpoint pathway protein selected from the group comprising OX40, ICOS, GITR, 4-1BB, CD40, and a tumour microenvironment affect component selected from the group comprising A2AR, HIF1, IDO and TLR.

5. A method according to any one of the preceding claims for predicting response to chemotherapy comprising an ADCC-capable antibody therapeutic.

6. A method according to any one of the preceding claims, in which the cancer is a HER2+ cancer, the ADCC capable molecule is an antibody that targets HER2, and in which the cancer cell line is a HER2+ cancer cell line.

7. A method according to Claim 6, in which the cancer is a HER2+ breast cancer, the ADCC-capable antibody targets HER2, and in which the cancer cell line is a HER2+ breast cancer cell line.

8. A method according to any one of Claims 1 to 5, in which the cancer is a HER2-low or HER2-negative cancer, preferably, in which the cancer is lung cancer or melanoma.

9. A method according to any one of the preceding claims, in which the ADCC-capable molecule is an ADCC-capable antibody, preferably, in which the ADCC-capable antibody is selected from trastuzumab, pertuzumab, or margetuximab.

10. A method according to any one of the preceding claims, in which the blood sample is a peripheral blood mononuclear cell (PBMC) fraction.

11. A method according to any one of the preceding claims, in which the reference ADCC response value is obtained by incubating a second aliquot of the patient's blood sample with the ADCC-capable molecule and the cancer cell line in the absence of the immune checkpoint protein modulator to provide a second sample; and determining an ADCC response of the second sample, wherein an ADCC response that is higher than the reference ADCC response is indicative that the patient will have a poor response to chemotherapy.

12. A method according to any preceding claim, in which the reference ADCC response value is obtained by incubating a second aliquot of the patient's blood sample with the ADCC capable molecule and the cancer cell line in the absence of the immune checkpoint protein modulator to provide a second sample; and determining an ADCC response of the second sample, wherein an ADCC response that is higher than the reference ADCC response is indicative that the patient will respond to chemotherapy comprising an immune checkpoint protein modulator, preferably in which the immune response modulator is an ICI.

13. A method according to Claim 1 for predicting response to a HER2-targetting ADCC capable antibody therapeutic in an individual with a HER2+ cancer, the method comprising the steps of: incubating a first aliquot of the patient's blood sample with a HER2-targetting ADCC capable molecule, the ICI, and a HER2+ cancer cell line for a period of time to provide a first sample;
determining an ADCC response of the first sample; incubating a second aliquot of the patient's blood sample with the HER2-targetting ADCC-capable molecule and the HER2+ cancer cell line in the absence of the ICI to provide a second sample; and determining an ADCC response of the second sample, and when the ADCC response of the first sample is higher than the ADCC response of the second sample, predicting that the patient will have a poor response to ADCC-capable antibody therapy.

14. A method according to Claim 13, in which the HER2+ cancer is selected from the group comprising HER2+ gastric, oesophageal, gastro-oesophageal, ovarian, bladder, cervical, salivary gland, endometrial, pancreatic, colo-rectal, prostate, testicular, melanoma, hepatocellular carcinoma, cholangiocarcinoma, head and neck, small intestine, gall bladder and non-small-cell lung cancer (NSCLC), preferably, in which the HER2+ cancer is HER2+ breast cancer.

15. A method of predicting response to chemotherapy in a patient with cancer, the method comprising the steps of:
incubating an immune checkpoint protein modulator, a cancer cell line, and a sample of the patient's blood, for a period of time,
determining direct cytotoxicity against the cancer cell line in the presence of an immune checkpoint protein modulator, comparing the direct cytotoxicity with a reference direct cytotoxicity in the absence of an immune checkpoint protein modulator, and predicting the patient's response to chemotherapy based on the comparison.

16. A method according to Claim 15, in which the reference direct cytotoxicity is determined by incubating the cancer cell line, and a second sample of the patient's blood, without the immune checkpoint protein modulator, for a period of time, and determining direct cytotoxicity against the cancer cell line to provide the reference direct cytotoxicity, and in which a direct cytotoxicity that is higher than the reference direct cytotoxicity is indicative that the patient will have a poor response to chemotherapy.

17. A method according to Claim 15 or 16, in which the reference direct cytotoxicity is obtained by incubating a second sample of the patient's blood sample with or without the immune checkpoint protein modulator for a period of time; and determining direct cytotoxicity of the second sample, wherein a direct cytotoxicity that is higher than the reference direct cytotoxicity is indicative that the patient will respond to therapy comprising an immune checkpoint protein modulator.

## Patentansprüche

1. Verfahren zur Vorhersage der Reaktion auf Chemotherapie bei einem Patienten mit Krebs, wobei das Verfahren die folgenden Schritte umfasst:
Inkubieren eines ADCC-fähigen Moleküls, eines Immun-Checkpoint-Protein-Modulators, einer Krebszelllinie, die ein Ziel des ADCC-fähigen Moleküls exprimiert, und einer Probe des Patientenbluts für eine Zeitdauer;
Bestimmen einer ADCC-Reaktion gegen die Krebszelllinie in Gegenwart des Immun-Checkpoint-Protein-Modulators,
Vergleichen der ADCC-Reaktion mit einer ADCC-Referenzreaktion bei Abwesenheit eines Immun-Checkpoint-Protein-Modulators, und
Vorhersagen der Patientenreaktion auf Chemotherapie basierend auf dem Vergleich, wobei sich ein ADCC-fähiges Molekül auf ein Molekül bezieht, das zur Bindung eines Zelloberflächenproteins oder Antigens, wie zum Beispiel eines Rezeptors, auf der Tumoroberfläche und Erzeugung einer antikörperabhängigen zellvermittelten Zytotoxizität (ADCC) oder antikörperabhängigen zellvermittelten Phagozytose (ADCP)-Reaktion gegen die Tumorzelle fähig ist, wodurch Immunzellen das Molekül binden und die Tumorzelle abtöten.

2. Verfahren nach Anspruch 1, in dem der Immun-Checkpoint-Protein-Modulator ein Immun-Checkpoint-Inhibitor (ICI) oder ein Immun-Checkpoint-Protein-Stimulator ist.

3. Verfahren nach Anspruch 2, in dem der Immun-Checkpoint-Protein-Modulator ein ICI ist, bevorzugt in dem der ICI aus Pembrolizumab, Nivolumab, Atezolizumab und Durvalumab, Avelumab und Ipilimumab ausgewählt ist.

4. Verfahren nach einem der vorstehenden Ansprüche, in dem das Checkpoint-Protein aus der Gruppe ausgewählt ist, die Folgende umfasst: Lymphozyten-Aktivierungs-Gen 3 (LAG-3), T-Zell-5-Immunglobulin-3 (TIM-3), T-Zell-Immunglobulin, ITIM-Domäne (TIGIT), V-Domäne-Ig-Suppressor der T-Zell-Aktivierung (VISTA), B7-H3, ein Protein des immunstimulatorischen Checkpoint-Wegs, das aus der Gruppe ausgewählt ist, die OX40, ICOS, GITR, 4-1BB, CD40 umfasst, und eine die Mikroumgebung eines Tumors beeinflussende Komponente, die aus der Gruppe ausgewählt ist, die A2AR, HIF1, IDO und TLR umfasst.

5. Verfahren nach einem der vorstehenden Ansprüche für die Vorhersage der Reaktion auf Chemotherapie, die ein ADCC-fähiges Antikörpertherapeutikum umfasst.

6. Verfahren nach einem der vorstehenden Ansprüche, in dem der Krebs ein HER2+-Krebs ist, das ADCC-fähige Molekül ein Antikörper ist, der auf HER2 abzielt, und in dem die Krebszelllinie eine HER2+-Krebszelllinie ist.

7. Verfahren nach Anspruch 6, in dem der Krebs ein HER2+-Brustkrebs ist, der ADCC-fähige Antikörper auf HER2 abzielt und in dem die Krebszelllinie eine HER2+-Brustkrebszelllinie ist.

8. Verfahren nach einem der Ansprüche 1 bis 5, in dem der Krebs ein HER2-niedriger oder HER2-negativer Krebs ist, bevorzugt in dem der Krebs Lungenkrebs oder Melanom ist.

9. Verfahren nach einem der vorstehenden Ansprüche, in dem das ADCC-fähige Molekül ein ADCC-fähiger Antikörper ist, bevorzugt in dem der ADCC-fähige Antikörper aus Trastuzumab, Pertuzumab oder Margetuximab ausgewählt ist.

10. Verfahren nach einem der vorstehenden Ansprüche, in dem die Blutprobe eine Fraktion von peripheren mononukleären Blutzellen (PBMC) ist.

11. Verfahren nach einem der vorstehenden Ansprüche, in dem der ADCC-Referenzreaktionswert durch Folgendes erhalten wird: Inkubieren eines zweiten Aliquoten der Patientenblutprobe mit dem ADCC-fähigen Molekül und der Krebszelllinie bei Abwesenheit des Immun-Checkpoint-Protein-Modulators, um eine zweite Probe bereitzustellen; und Bestimmen einer ADCC-Reaktion der zweiten Probe, wobei eine ADCC-Reaktion, die höher ist als die ADCC-Referenzreaktion, anzeigt, dass der Patient eine schlechte Reaktion auf Chemotherapie haben wird.

12. Verfahren nach einem vorstehenden Anspruch, in dem der ADCC-Referenzreaktionswert durch Folgendes erhalten wird: Inkubieren eines zweiten Aliquoten der Patientenblutprobe mit dem ADCC-fähigen Molekül und der Krebszelllinie bei Abwesenheit des Immun-Checkpoint-Protein-Modulators, um eine zweite Probe bereitzustellen; und Bestimmen einer ADCC-Reaktion der zweiten Probe, wobei eine ADCC-Reaktion, die höher ist als die ADCC-Referenzreaktion, anzeigt, dass der Patient auf eine Chemotherapie reagieren wird, die einen Immun-Checkpoint-Protein-Modulator umfasst, bevorzugt in dem der Immunreaktionsmodulator ein ICI ist.

13. Verfahren nach Anspruch 1 zur Vorhersage der Reaktion auf ein auf HER2 abzielendes ADCC-fähiges Antikörpertherapeutikum bei einem Individuum mit einem HER2+-Krebs, wobei das Verfahren die folgenden Schritte umfasst: Inkubieren eines ersten Aliquoten der Patientenblutprobe mit einem auf HER2 abzielenden ADCC-fähigen Molekül, dem ICI und einer HER2+-Krebszelllinie für eine Zeitdauer, um eine erste Probe bereitzustellen;
Bestimmen einer ADCC-Reaktion der ersten Probe, Inkubieren eines zweiten Aliquoten der Patientenblutprobe mit dem auf HER2 abzielenden ADCC-fähigen Molekül und der HER2+-Krebszelllinie bei Abwesenheit des ICI, um eine zweite Probe bereitzustellen; und Bestimmen einer ADCC-Reaktion der zweiten Probe und, wenn die ADCC-Reaktion der ersten Probe höher ist als die ADCC-Reaktion der zweiten Probe, Vorhersagen, dass der Patient eine schlechte Reaktion auf ADCC-fähige Antikörpertherapie haben wird.

14. Verfahren nach Anspruch 13, in dem der HER2+-Krebs aus der Gruppe ausgewählt ist, die Folgende umfasst: HER2+-Magen-, -Speiseröhren-, - gastroösophagealen, -Eierstock-, -Blasen-, -Gebärmutterhals-, -Speicheldrüsen-, - Endometrium-, -Bauchspeicheldrüsen-, -kolorektalen, -Prostata-, -Hoden-, -Melanom-, - Leberzellkarzinom, -Gallengangkarzinom, -Kopf-Hals-, -Dünndarm-, -Gallenblasen- und -nicht-kleinzelligen Lungenkrebs (NSCLC), bevorzugt in dem der HER2+-Krebs HER2+-Brustkrebs ist.

15. Verfahren zur Vorhersage der Reaktion auf Chemotherapie bei einem Patienten mit Krebs, wobei das Verfahren die folgenden Schritte umfasst:
Inkubieren eines Immun-Checkpoint-Protein-Modulators, einer Krebszelllinie und einer Probe des Patientenbluts für eine Zeitdauer;
Bestimmen von direkter Zytotoxizität gegen die Krebszelllinie in Gegenwart eines Immun-Checkpoint-Protein-Modulators, Vergleichen der direkten Zytotoxizität mit einer direkten Referenzzytotoxizität bei Abwesenheit eines Immun-Checkpoint-Protein-Modulators und Vorhersagen der Patientenreaktion auf Chemotherapie basierend auf dem Vergleich.

16. Verfahren nach Anspruch 15, in dem die direkte Referenzzytotoxizität durch Folgendes bestimmt wird: Inkubieren der Krebszelllinie und einer zweiten Probe des Patientenbluts, ohne den Immun-Checkpoint-Protein-Modulator, für eine Zeitdauer und Bestimmen von direkter Zytotoxizität gegen die Krebszelllinie, um die direkte Referenzzytotoxizität bereitzustellen, und, in dem eine direkte Zytotoxizität, die höher ist als die direkte Referenzzytotoxizität, anzeigt, dass der Patient eine schlechte Reaktion auf Chemotherapie haben wird.

17. Verfahren nach Anspruch 15 oder 16, in dem die direkte Referenzzytotoxizität durch Folgendes erhalten wird: Inkubieren einer zweiten Probe der Patientenblutprobe mit oder ohne dem Immun-Checkpoint-Protein-Modulator für eine Zeitdauer; und Bestimmen der direkten Zytotoxizität der zweiten Probe, wobei eine direkte Zytotoxizität, die höher ist als die direkte Referenzzytotoxizität, anzeigt, dass der Patient auf eine Therapie reagieren wird, die einen Immun-Checkpoint-Protein-Modulator umfasst.

## Revendications

1. Méthode de prédiction de la réponse à une chimiothérapie chez un patient souffrant d'un cancer, la méthode comprenant les étapes :
d'incubation d'une molécule capable d'ADCC, d'un modulateur de protéine de point de contrôle immunitaire, d'une lignée de cellules cancéreuses qui exprime une cible de la molécule capable d'ADCC, et d'un échantillon du sang du patient, pendant une période de temps ;
de détermination d'une réponse de type ADCC contre la lignée de cellules cancéreuses en présence dudit modulateur de protéine de point de contrôle immunitaire ;
de comparaison de la réponse de type ADCC avec une réponse de type ADCC de référence en l'absence d'un modulateur de protéine de point de contrôle immunitaire ; et
de prédiction de la réponse du patient vis-à-vis d'une chimiothérapie sur la base de la comparaison, où une molécule capable d'ADCC désigne une molécule qui est capable de se lier à une protéine de surface cellulaire, ou à un antigène, tel qu'un récepteur, à la surface de la tumeur, et de générer une cytotoxicité à médiation cellulaire dépendante des anticorps (ADCC), ou une réponse de phagocytose à médiation cellulaire dépendante des anticorps (ADCP), contre la cellule tumorale de sorte que les cellules immunitaires se lient à la molécule et tuent la cellule tumorale.

2. Méthode selon la revendication 1, le modulateur de protéine de point de contrôle immunitaire étant un inhibiteur de points de contrôle immunitaire (ICI) ou un stimulateur de protéines de points de contrôle immunitaire.

3. Méthode selon la revendication 2, le modulateur de protéine de point de contrôle immunitaire étant un ICI, de préférence où l'ICI est choisi parmi le pembrolizumab, le nivolumab, l'atézolizumab et le durvalumab, l'avélumab et l'ipilimumab.

4. Méthode selon l'une quelconque des revendications précédentes, la protéine de point de contrôle étant choisie dans le groupe comprenant le gène-3 d'activation des lymphocytes (LAG-3), l'immunoglobuline-3 des lymphocytes T-5 (TIM-3), le domaine d'immunoglobuline de lymphocyte T et ITIM (TIGIT), le suppresseur de type immunoglobuline à domaine V de l'activation des lymphocytes T (VISTA), B7-H3, une protéine de la voie des points de contrôle de stimulation immunitaire choisie dans le groupe comprenant OX40, ICOS, GITR, 4-1BB, CD40, et un composant affectant le microenvironnement tumoral choisi dans le groupe comprenant A2AR, HIF1, IDO et TLR.

5. Méthode selon l'une quelconque des revendications précédentes, destinée à la prédiction de la réponse à une chimiothérapie, comprenant une substance thérapeutique à base d'un anticorps capable d'ADCC.

6. Méthode selon l'une quelconque des revendications précédentes, le cancer étant un cancer HER2+, la molécule capable d'ADCC est un anticorps qui cible HER2, et où la lignée de cellules cancéreuses est une lignée de cellules cancéreuses HER2+.

7. Méthode selon la revendication 6, le cancer étant un cancer du sein HER2+, l'anticorps capable d'ADCC cible HER2, et où la lignée de cellules cancéreuses est une lignée de cellules du cancer du sein HER2+.

8. Méthode selon l'une quelconque des revendications 1 à 5, le cancer étant un cancer HER2-low ou HER2-négatif, de préférence où le cancer est un cancer du poumon ou un mélanome.

9. Méthode selon l'une quelconque des revendications précédentes, la molécule capable d'ADCC étant un anticorps capable d'ADCC, de préférence où l'anticorps capable d'ADCC est choisi parmi le trastuzumab, le pertuzumab ou le margétuximab.

10. Méthode selon l'une quelconque des revendications précédentes, l'échantillon de sang étant une fraction de cellules mononucléées du sang périphérique (PBMC).

11. Méthode selon l'une quelconque des revendications précédentes, la valeur de la réponse de type ADCC de référence étant obtenue par incubation d'une deuxième aliquote de l'échantillon de sang du patient avec la molécule capable d'ADCC et la lignée de cellules cancéreuses en l'absence du modulateur de protéine de point de contrôle immunitaire afin de fournir un deuxième échantillon ; et la détermination d'une réponse de type ADCC du deuxième échantillon, où une réponse de type ADCC qui est supérieure à la réponse de type ADCC de référence indique que le patient aura une faible réponse à une chimiothérapie.

12. Méthode selon l'une quelconque des revendications précédentes, la valeur de la réponse de type ADCC de référence étant obtenue en incubant une deuxième aliquote de l'échantillon de sang du patient avec la molécule capable d'ADCC et la lignée de cellules cancéreuses en l'absence du modulateur de protéine de point de contrôle immunitaire pour fournir un deuxième échantillon ; et la détermination d'une réponse de type ADCC du deuxième échantillon, où une réponse de type ADCC qui est supérieure à la réponse de type ADCC de référence indique que le patient répondra à une chimiothérapie comprenant un modulateur de protéine de point de contrôle immunitaire, de préférence où le modulateur de réponse immunitaire est un ICI.

13. Méthode selon la revendication 1, destinée à la prédiction de la réponse à une substance thérapeutique à base d'un anticorps capable d'ADCC et ciblant HER2 chez un individu souffrant d'un cancer HER2+, la méthode comprenant les étapes : d'incubation d'une première aliquote de l'échantillon de sang du patient avec une molécule capable d'ADCC ciblant HER2, l'ICI, et une lignée de cellules cancéreuses HER2+ pendant une période de temps afin de fournir un premier échantillon ;
de détermination d'une réponse de type ADCC du premier échantillon ;
d'incubation d'une deuxième aliquote de l'échantillon de sang du patient avec la molécule capable d'ADCC ciblant HER2 et la lignée de cellules cancéreuses HER2+ en l'absence de l'ICI afin de fournir un deuxième échantillon ; et de détermination d'une réponse de type ADCC du deuxième échantillon, et lorsque la réponse de type ADCC du premier échantillon est supérieure à la réponse de type ADCC du deuxième échantillon, de prédiction que le patient aura une faible réponse à une thérapie par anticorps capable d'ADCC.

14. Méthode selon la revendication 13, le cancer HER2+ étant choisi dans le groupe comprenant le cancer HER2+ gastrique, oesophagien, gastro-oesophagien, ovarien, de la vessie, cervical, des glandes salivaires, endométrial, pancréatique, colo-rectal, de la prostate, testiculaire, un mélanome, un carcinome hépatocellulaire, un cholangiocarcinome, un cancer de la tête et cou, de l'intestin grêle, de la vésicule biliaire et du poumon non à petites cellules (NSCLC), de préférence où le cancer HER2+ est un cancer du sein HER2+.

15. Méthode destinée à la prédiction de la réponse à une chimiothérapie chez un patient atteint d'un cancer, la méthode comprenant les étapes :
d'incubation d'un modulateur de protéine de point de contrôle immunitaire, d'une lignée de cellules cancéreuses et d'un échantillon de sang du patient, pendant une période de temps,
de détermination de la cytotoxicité directe contre la lignée de cellules cancéreuses en présence d'un modulateur de protéine de point de contrôle immunitaire, de comparaison de la cytotoxicité directe à une cytotoxicité directe de référence en l'absence d'un modulateur de protéine de point de contrôle immunitaire, et de prédiction de la réponse du patient à la chimiothérapie sur la base de la comparaison.

16. Méthode selon la revendication 15, la cytotoxicité directe de référence étant déterminée par l'incubation de la lignée de cellules cancéreuses et d'un deuxième échantillon du sang du patient, sans le modulateur de protéine de point de contrôle immunitaire, pendant une période de temps, et la détermination de la cytotoxicité directe contre la lignée de cellules cancéreuses pour fournir la cytotoxicité directe de référence, et où une cytotoxicité directe qui est supérieure à la cytotoxicité directe de référence indique que le patient aura une faible réponse à une chimiothérapie.

17. Méthode selon la revendication 15 ou 16, la cytotoxicité directe de référence étant obtenue par l'incubation d'un deuxième échantillon de l'échantillon de sang du patient avec ou sans le modulateur de protéine de point de contrôle immunitaire pendant une période de temps ; et la détermination de la cytotoxicité directe du deuxième échantillon, où une cytotoxicité directe qui est supérieure à la cytotoxicité directe de référence indique que le patient répondra à une thérapie comprenant un modulateur de protéine de point de contrôle immunitaire.
